# EUROPEAN PATENT SPECIFICATION

(11) **EP 0 325 762 B1**
(45) Date of publication and mention of the grant of the patent: **22.03.1995**
(21) Application number: 88121087.6
(22) Date of filing: 16.12.1988
(51) Int. Cl.: C07D 257/02, A61K 49/00, G01N 24/08

(54) **Macrocyclic chelating agents and chelates thereof**
Makrocyclische Chelatisierungsmittel und daraus hergestellte Chelate
Agents macrocycliques de chélation et chélates préparés à partir de ceux-ci

(30) Priority: 24.12.1987 IT 2321787
(43) Date of publication of application: 02.08.1989
(73) Proprietor: BRACCO S.p.A., 20134 Milano (IT)
(72) Inventor: Felder, Ernst, I-20134 Milano (IT); Musu, Carlo, I-20134 Milano (IT); Fumagalli, Luciano, I-20134 Milano (IT); Uggeri, Fulvio, I-20134 Milano (IT)
(74) Representative: Minoja, Fabrizio

(56) References cited:
- EP-A- 0 124 766
- EP-A- 0 232 751
- EP-A- 0 255 471
- WO-A-87/06229
- DE-A- 3 401 052
- US-A- 4 639 365
- Intern. J. of Radiation/ Applications and Instrumentation, part B, vol. 15, no. 1, 1988, Marsh Barton, Exeter, GB; J.F. Desreux et al.
- Angewandte Chemie, vol. 88, no. 22, 1976, p. 760; H. Stetter et al.

## Description

The present invention relates to novel macrocyclic chelating agents deriving from 1,4,7,10-tetraazacyclododecane of general formula I 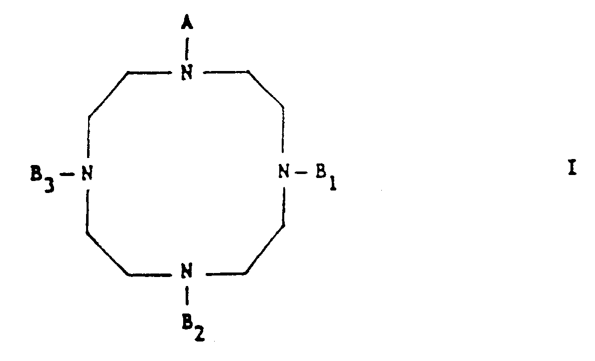
wherein
- A: is a group of formula 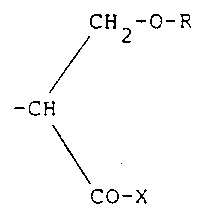
in which
- R: is H or a C₁-C₅ straight or branched alkyl group, or a benzyl group which can be mono- or poly-substituted on the aromatic ring by halogen, hydroxy, carboxy, carbamoyl, alkoxycarbonyl, sulphamoyl, lower alkyl, lower hydroxyalkyl, amino, acylamino, acyl, hydroxyacyl groups, or a group of formula H(OCH₂CH₂)₁₋₄-, Me(OCH₂CH₂)₁₋₄-, or Et(OCH₂CH₂)₁₋₄-,
- X: is a O-R₁ group in which R₁ is H or a C₁-C₅ alkyl, hydroxyalkyl, alkoxyalkyl, alkoxyhydroxyalkyl group, or a polyoxaalkyl group having 1 to 15 oxygen atoms and 3 to 45 carbon atoms, or X is a -NR₂R₃ group in which R₂ and R₃, which can be the same or different, are hydrogen, C₁-C₆ alkyl, hydroxyalkyl, alkoxyalkyl or alkoxyhydroxyalkyl groups having up to 5 hydroxy groups
and
B₁, B₂ and B₃, which can be the same or different, have the same meaning as A or they are H or a group of formula 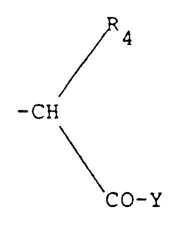
in which
- R₄: is H or a C₁-C₅ straight or branched alkyl group,
- Y: is a O-R₅ group in which R₅ is H or a C₁-C₅ alkyl, hydroxyalkyl, alkoxyalkyl, alkoxyhydroxyalkyl group, or a polyoxaalkyl group having 1 to 15 oxygen atoms and 3 to 45 carbon atoms, or Y is a -NR₆R₇ group in which R₆ and R₇, which can be the same or different, are H or C₁-C₆ alkyl, hydroxyalkyl, alkoxyalkyl or alkoxyhydroxyalkyl groups having up to 5 hydroxy groups,
said derivatives being, if necessary, salified with suitable organic or inorganic bases,
and the complex salts of the abovesaid chelating agents with suitable metal ions in the acid, basic or neutral form or, if necessary, neutralized with inorganic or organic acid or base ions, and eventually chemically conjugated with macromolecules or incorporated in suitable carriers.

The present invention also relates to the preparation of compounds of general formula I and of the complex salts thereof, to their uses and, when indicated, to the pharmaceutical and diagnostic compositions thereof.

The chelating compounds of the present invention and the complex salts thereof can have a wide range of applications. No limiting examples of use of said chelating agents are the recovery, separation, selective extraction of metal ions even at very low concentrations, their therapeutical use as detoxifying agents in cases of inadvertent bodily incorporation of metals or radioisotopes, their use as ion carriers, or the other ones apparent to those skilled in the art. In such uses the chelating agents may be used directly or often they have been bonded covalently or non-covalently to macromolecules or insoluble surfaces or have been otherwise incorporated into structures that can carry them to specific sites.

In particular the complex salts of the chelating agents of formula I with the metal ions of the elements with atomic numbers of 20 to 31, 39, 42, 43, 44, 49 or 57 to 83 and, optionally, salified by physiologically biocompatible ions of organic or inorganic acids or organic or inorganic bases or aminoacids, are surprisingly suitable for use as contrast agents in medical diagnosis in nuclear medicine and in N.M.R., E.S.R., X-ray and/or ultrasonic diagnosis.

Said derivatives, for the purpose of optimal diagnostic use, can also be bound or incorporated covalently or non-covalently into biomolecules, macromolecules or molecular aggregates characterized in that they can selectively concentrate in the organ or in the tissue under examination.

The imaging of internal structures of living subjects is becoming more and more relevant in medical diagnosis.

Among the most recent techniques, the use of radioisotopes as internal tracers in the organism should be mentioned.

One of the biggest problems connected with the use of radioisotopes is their selectivity of distribution, while another important aspect is their excretion in an acceptable time.

Another imaging technique concerns with the use of ultrasounds to measure the difference in the reflections at the interfaces between tissues of different density.

The administration of a suitable amount of a dense non-radioactive element or metal ion can give such a difference in reflectivity that can emphasize even small otherwise non detectable lesions.

A third diagnostic technique uses nuclear magnetic resonance to create internal images of the human body. In this field, the development of contrast agents is of particular importance for the following reasons:
a) to improve the specificity of the diagnosis,
b) to identify at an earlier stage small lesions,
c) to more precisely define the extension of a tumoral mass,
d) to improve the signal to noise ratio and to shorten the time of acquisition of the images, allowing also better use of the instruments,
e) to increase the contrast between those contiguous areas (for instance abdominal or pelvic) where it is particularly difficult to obtain well defined images,
f) to obtain good informations on blood flow and on tissue perfusion.

As far as regards N.M.R. diagnosis, contrast media containing paramagnetic complex salts of lanthanides and transition metals have already been claimed for instance in EP 71564 and in US-Pat. 4,639,365, and in patent applications DE 3401052, EP-A 135125, EP-A 130934, DE 3324236, EP-A 124766, EP-A 165728, WO 87/02893, EP-A 230893.

However all the till now developed contrast agents for N.M.R. present some problems as far as regards their capacity of influencing the relaxation time of the atomic nuclei involved, their often insufficient selectivity in bonding the metal ion, their stability, their selectivity for the organ under examination, or their biological tolerability. The tendency of many complexes to exchange the central metal ion with trace-metals essential to the organism or with ions, for example Ca⁽²⁺⁾, which in vivo are present in relatively great amounts (see on the subject P.M. May "The present status of chelating agents in Medicine", page 233), further limits their possibilities of use. In fact, in case of insufficient stability of the complex, the organism may be deprived of trace-metals of vital importance and receive undesired and toxic heavy metals such as Gd, Eu or Dy. Although it is true that the toxicity of the complex is often, but not always, lower than the one of the free paramagnetic ion, it is also true that the complexation usually brings a decrease of the magnetic relaxation efficacy, responsible for some contrasting effects.

Several unsolved problems in connection with an optimal contrast agent therefore still remain, chiefly concerning: a strong effect on the relaxation time of the relevant nuclei, a high stability of the complex both in solution and in the organism, an adequate water solubility, a specificity of distribution in the various parts of the organism, a suitable rate of elimination from the involved organ and tissue.

One of the most studied paramagnetic metal ions is Gd⁽³⁺⁾, in particular when complexed with the chelating agent diethylenetriamino-pentaacetic acid (DTPA) (Runge et al. (1983) Am. J. Radiol. V 141, p. 1209 and Weinman et al. (1984) Am. J. Radiol. V 142, p. 619). Said complex salified with D(-)N-methyl-glucamine is considered at the moment one of the most satisfying from the point of view of activity, toxicity and of its use in general.

However, in spite of these positive features, this compound cannot yet be considered fully respondent to the characteristics of an optimal contrast agent for various reasons, among which for instance the fact that Gd-DTPA/N-Methyl-D-glucamine is too quickly removed from the blood stream and from the lesions of the tissues under examination. This reduces the time available for obtaining images significant from diagnostic point of view. Moreover the diffusion of the contrast agent between the healthy part and the diseased one is often so fast that the contrast between the two regions can be too weak.

To overcome these difficulties, the problem has been approached in many ways among which the most interesting are:
a) Other chelating agents have been studied, in particular macrocyclic ones, of which the most effective proved to be 1,4,7,10-tetraazacyclododecane-N,N',N'',N'''-tetraacetic acid (DOTA). However its complexes continue to present problems analogous to the ones of DTPA.
b) Gadolinium and its chelating agents have been chemically conjugated to macromolecules such as, for instance, proteins (albumin, etc.), immunoglobulins, or to cellulose or other polymeric matrices. On the one hand this generally improved the relaxivity of Gd, but on the other hand it was necessarily accompanied by a sub-optimal dosage, because of limitations in solubility, toxicity and the substitution density of the macromolecules. Furthermore, when one of the ligand sites of the chelating agents is used to form the chemical bond with the macromolecule, there is also normally a reduction in the stability of the resulting complex.

EP-A-272751, Int. J. Rad/Appl. and Instr. PT 13 15(1), 1988, 9-15 and EP-A-255471 disclose 1,4,7,10-tetraazadodecanes substituted in the 1-, 4- and 7-positions by acetic acid residues which are α-substituted by alkyl groups. These compounds have a good metal chelating ability but lower relaxivities values in comparison with the compound of the invention.

The chelating agents of formula I have shown an excellent scavenging capacity for metal ions even in very diluted solutions. A significant example of said property is the capacity to capture the Cu⁽²⁺⁾ ion from its aqueous solutions by 2-[1,4,7,10-tetraaza-4,7,10-tri(carboxymethyl)cyclododeca-ne-1-yl]-3-benzyloxypropionic acid, the method of synthesis of which is reported in examples 2 and 3. With regards to their use in diagnosis, metal complexes with the chelating agents object of the present invention have proved surprisingly satisfying for instance with respect to the requirements for an N.M.R. contrast agent. Among the complexes particular importance is to be given to the complexes of Gd⁽³⁺⁾, which distinguish themselves for excellent stability, relaxivity and selectivity for the organ or tissue under examination.

These compounds have a wide field of application, allowing administration by intravasal route (for example intravenous, intraarterial, intracoronaric, intraventricular, etc.), as well as intrathecal, intraperitoneal, intralymphatical, intracavital and intraparenchymal routes. Both the soluble and the poorly soluble compounds are suitable for oral or enteral administration, and therefore of particular usefulness for visualization of the gastrointestinal tract. For parenteral administration they are preferably formulated as a sterile aqueous suspension or solution, whose pH can range for instance from 6.0 to 8.5. Said sterile aqueous suspensions or solutions can be administered in concentrations varying from 0.002 to 1.0 molar.

Said formulations, can also be lyophilized and supplied as such, to be reconstituted at the moment of their use. For gastrointestinal use or for injection in body cavities said agents can be formulated as a suspension or a solution containing additives suitable for instance to increase viscosity.

For oral administration they can be formulated according to preparation methods commonly used in pharmaceutical technology, optionally also as a coated formulation so as to have additional protection against the acid pH of the stomach, preventing in that way the release of the chelated metal ions which takes place in particular at the pH values typical of gastric juices. Other excipients, such as sweetening or flavouring agents, can be added according to known pharmaceutical formulation techniques.

Suspensions or solutions of complex salts can also be formulated as aerosols to be used in aerosol-bronchography.

Some of the complex compounds of the invention have a surprising organ specificity, in that they particularly concentrate in the liver, in the spleen or, after intralymphatic, intraparenchymal, intramuscular or subcutaneous application, in the lymphatic vases and in the lymph nodes. The resulting contrast between the organ under examination and adjacent tissues permits improved imaging of said organ by N.M.R.

With regard to their use in diagnosis, metal complexes of the chelating agents object of the present invention can also be used as contrast agents in nuclear medicine and for electron spin resonance or echographic analyses.

In these cases however the metal central ions in the chelated complexes are, respectively, a radioisotope for example ⁵¹Cr, ⁶⁸Ga, ¹¹¹In, ^{99m}Tc, ¹⁴⁰La, ¹⁶⁸Yb or a non-radioisotope able to alter, owing to the density of its solutions, the speed of the ultrasonic waves transmitted and reflected.

In the compounds of general formula I, A is preferably a β-hydroxy-α-propionic, β-methoxy-α-propionic or β-benzyloxy-α-propionic group, optionally esterified or preferably substituted by an amide residue which can be free, mono- or bi-substituted by alkyl, hydroxyalkyl, alkoxyalkyl or alkoxyhydroxyalkyl groups.

R can preferably be H or a straight or branched alkyl group, such as a methyl, ethyl, propyl, isopropyl, butyl, isobutyl group or a benzyl or a substituted benzyl group as defined in formula I.

R can also be an acyl or hydroxyacyl group.

R can also be a polyoxaethylene group of formula H(OHCH₂CH₂)₂₋₄-, Me(OCH₂CH₂)₂₋₄-, o Et(OCH₂CH₂)₂₋₄-.

X can be a hydroxy group or also a -O-R₁ group, wherein R₁ is as defined in formula I.

Non-limiting examples of R₁ are the following: methyl, ethyl, isopropyl, 2-hydroxyethyl, 2-hydroxypropyl, 1,3-dihydroxyisopropyl, polyoxaalkyl groups.

X can preferably be also an hydroxyalkylamino group of formula -NR₂R₃, in which R₂ and R₃ are as defined in formula I. Non-limiting examples of said groups are the following ones:
amino-, 2-hydroxyethylamino-, 2-hydroxypropylamino-, 2,3-dihydroxypropylamino-, 1,3-dihydroxyisopropylamino-, 1,3-dihydroxy-2-methyl-isopropylamino-, 2,3,4-trihydroxy-1-butylamino-, 1,3,4-trihydroxy-2-butylamino-, 1,3-dihydroxy-2-hydroxymethyl-isopropylamino-, N-methyl-N-2-hydroxyethylamino-, N-methyl-N-2,3-dihydroxypropylamino-, N-methyl-N-1,3-dihydroxyisopropylamino-, N-methyl-N-2,3,4,5,6-pentahydroxyhexylamino-, N-2-hydroxyethyl-N-2,3-dihydroxypropylamino-, N-2-hydroxyethyl-N-1,3-dihydroxyisopropylamino-, N,N-bis-(2-hydroxyethyl)amino-, N,N-bis-(2,3-dihydroxypropyl)amino-, N,N-bis-(1,3-dihydroxyisopropyl)amino groups.

In compounds of general formula I, the B₁, B₂, B₃ groups preferably are an acetic or an α-propionic group, eventually esterified or substituted by an amido residue which can be in the free form or mono- or bi-substituted by alkyl, hydroxyalkyl, alkoxyalkyl or alkoxyhydroxyalkyl groups.

R₄ can preferably be hydrogen or straight or branched lower alkyl, preferably methyl.

Non-limiting examples for R₄ are the following: hydrogen, methyl, straight or branched propyl, butyl and pentyl groups, as defined in formula I.

Y can preferably be a hydroxy group or a -O-R₅ group, in which R₅ has the above defined meanings of formula I.

Non limiting examples of R₅ are the following: methyl, ethyl, isopropyl, 2-hydroxyethyl, 2-hydroxypropyl, 1,3-dihydroxyisopropyl, polyoxaalkyl groups.

Y can preferably be also a hydroxyalkylamino group of formula -NR₆R₇ in which R₆ and R₇ have the above mentioned meanings of formula I.

Non-limiting examples of said groups are the following ones:
amino, 2-hydroxyethylamino-, 2-hydroxypropylamino-, 2,3-dihydroxypropylamino- 1,3-dihydroxyisopropylamino-, 1,3-dihydroxy-2-methyl-isopropylamino-, 2,3,4-trihydroxy-1-butylamino-, 1,3,4-trihydroxy-2-butylamino-, 1,3-dihydroxy-2-hydroxymethyl-isopropylamino-, N-methyl-N-2-hydroxyethylamino-, N-methyl-N-2,3-dihydroxypropylamino-, N-methyl-N-1,3-dihydroxyisopropylamino-, N-methyl-N-2,3,4,5,6-pentahydroxyhexylamino-, N-2-hydroxyethyl-N-2,3-dihydroxypropylamino-, N-2-hydroxyethyl-N-1,3-dihydroxyisopropylamino-, N,N-bis-(2-hydroxyethyl)amino-, N,N-bis-(2,3-dihydroxypropyl)amino-, N,N-bis-(1,3-dihydroxyisopropyl)amino groups.

Metal ions suited to form complex salts with the chelating agents of general formula I are mainly the di- or trivalent ions of the elements having atomic numbers ranging from 20 to 31, 39, 42, 43, 44, 49, or from 57 to 83 and particularly preferred are Fe⁽²⁺⁾, Fe⁽³⁺⁾, Cu⁽²⁺⁾, Cr⁽³⁺⁾, Gd⁽³⁺⁾, Eu⁽³⁺⁾, Dy⁽³⁺⁾ or Mn⁽²⁺⁾.

Among the metal radioisotopes, particularly preferred are ⁵¹Cr, ⁶⁸Ga, ¹¹¹In, ^{99m}Tc, ¹⁴⁰La, ¹⁶⁸Yb.

Preferred inorganic acid anions comprise ions such as chlorides, bromides, iodides or other ions such as sulfate. Preferred organic acid anions comprise ions of acids which are generally pharmaceutically used to salify basic substances, such as acetate, succinate, citrate, fumarate, maleate.

Preferred inorganic base cations comprise alkali metal ions, such as lithium, potassium and sodium, the latter being particularly preferred.

Preferred organic base cations comprise primary, secondary and tertiary amines, such as ethanolamine, diethanolamine, morpholine, glucamine, N,N-dimethylglucamine and N-methylglucamine, the latter being preferred.

Preferred amino acid cations comprise, for example, those of lysine, arginine and ornithine.

Non-limiting examples of the macromolecules suited for conjugation with the chelate complexes of the invention are the following: biomolecules, such as hormones (insulin), prostaglandins, steroidal hormones, amino sugars, peptides, proteins (albumin, human serum albumin), lipids, antibodies such as monoclonal antibodies, polysaccharide chains.

The chelated complexes of the invention can also be incorporated into liposomes, used in form of mono- or multi-lamellar vescicles.

The chelating agents of general formula I and the complex salts thereof are preferably prepared by reacting 1,4,7,10-tetraazacyclododecane (II), prepared according to the method of Atkins et al. (JACS 96, 2268 (1974)), 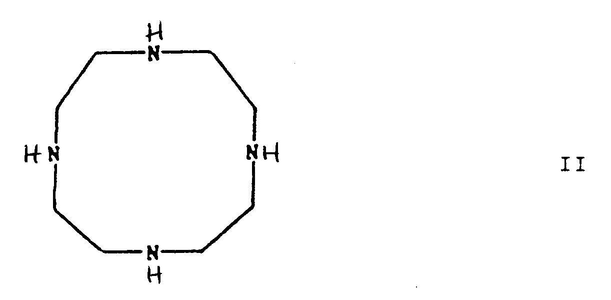
with the desired α-halo-propionyl derivative III 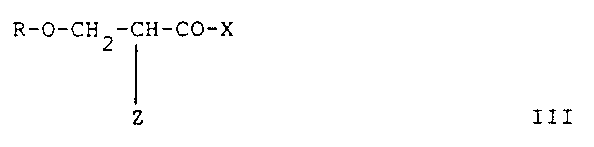
wherein Z is halogen and R and X have the above defined meanings, to give the addition product IV 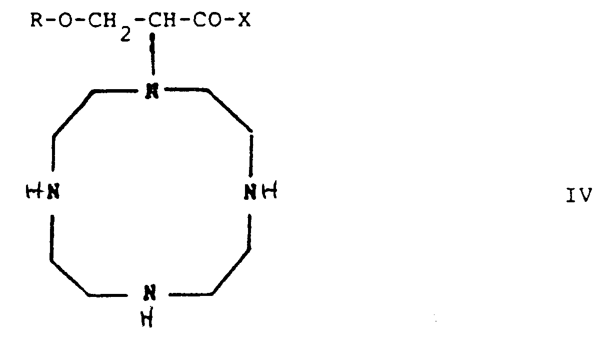
or the corresponding polysubstituted products on the nitrogen atoms of II, depending on the excess of III used. Compound IV can also be obtained, for example, by protecting diethylenetriamine V with a suitable protecting group P, wherein P can be, for example, a phthaloyl group or another appropriate protective group known in the literature (T.W. Greene: "Protective groups in organic synthesis"- 1980), 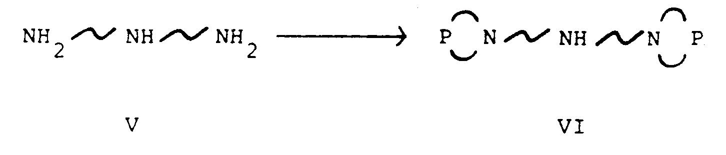
by alkylating the resulting compound VI with the proper halo-derivative III 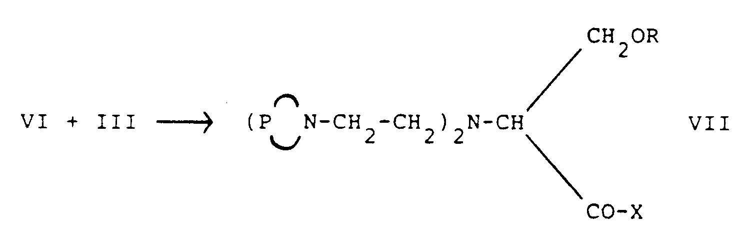
and by finally condensing the resulting product VII, after deprotection and subsequent tosylation, with tosyldiethanolamine.

Compound IV or the polysubstituted analogues thereof can in turn be subjected to condensation with the appropriate α-halo-acetic derivative VIII, or with a suitable precursor thereof (such as an ester or a nitrile), 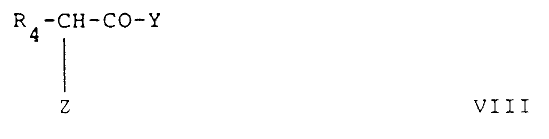
wherein Z is halogen and R₄ and Y have the above defined meanings, to give the desired chelating agents of general formula I.

Finally, chelation of the desired metal ion is obtained preferably by reacting the appropriate derivative of formula I with the stoichiometric amount of metal, in form of a salt or an oxide, possibly in the presence of the base or acid amounts necessary for neutralization.

Condensation of II with III is carried out preferably in water or in a dipolar aprotic organic solvent, such as dimethylformamide (DMF) or dimethylacetamide (DMAC) or in a mixture thereof, at a temperature from 30 to 150°C, preferably from 40 to 100°C.

Subsequent condensation of IV with VIII can be effected in an aqueous medium or in an organic solvent, in the presence of an appropriate inorganic or organic base, such as sodium hydroxide, potassium hydroxide, potassium carbonate or tetrabutylammonium hydroxide (TBAOH), at a pH ranging from 8 to 12, preferably from 9 to 11. The temperature can range from 40 to 100°C , preferably from 50 to 70°C.

Finally, preparation of the metal complex salt is preferably carried out in water or in an appropriate water-alcohol mixture, while the temperature can range from 25 to 100°C, preferably from 40 to 80°C.

The choice of the metal ion and, if necessary, of the neutralizing ion is strictly related to the use of the resulting complex.

### EXAMPLE 1

2-(1,4,7,10-tetraazacyclododecane-1-yl)-3-benzyloxypropionic acid, trihydrochloride.
A) Sodium 2-chloro-3-benzyloxypropionate
85 g of 2-chloro-3-benzyloxypropionic acid (0.396 mol) were suspended in 550 ml of water and neutralized to pH 7 with 10% sodium hydroxide. After stirring for 15 min, the resulting aqueous solution was washed with ethyl ether and evaporated to dryness under vacuum to give the desired compound.
90.6 g sodium 2-chloro-3-benzyloxypropionate (0.383 mol) were obtained.
Yield: 96.7%;

| Elemental analysis: | | | |
|---|---|---|---|
| % calc.: | C 50.75 ; | H 4.26 ; | Cl 14.98 |
| % found: | C 50.68 ; | H 4.33 ; | Cl 14.89 |

B) 2-(1,4,7,10-tetraazacyclododecane-1-yl)-3-benzyloxypropionic acid, trihydrochloride.
A suspension of 17.2 g of 1,4,7,10-tetraazacyclododecane (0.1 mol) and of 71 g of sodium 2-chloro-3-benzyloxypropionate (0.3 mol) in 70 ml of water was heated to 50°C for 24 h. The resulting solution was diluted to 400 ml with water, dropped into 200 ml of 2N HCl, extracted several times with methylene chloride and then was evaporated to dryness under vacuum.
The crude residue was taken up into 400 ml of water and adsorbed on amberlite IR 120, from which it was eluted by means of 5N sodium hydroxide. By concentration of the basic eluate 29 g of a residue were obtained, which were dissolved in 400 ml of absolute ethanol at 60°C; the solution was acidified with 200 ml of 6N HCl/EtOH and the resulting precipitate was stirred at 60°C for 1 h. After cooling, the solid was filtered and dried to give the desired compound.
33.5 g of 2-(1,4,7,10-tetraazacyclododecane-1-yl)-3-benzyloxypropionic acid, trihydrochloride (0.0729 mol) were obtained.
Yield: 72.9% m.p. 221-224°C
Titres:
(NaOH) : 96.9%
(AgNO₃): 99.0%

| Elemental analysis: | | | | |
|---|---|---|---|---|
| % calc.: | C 47.01 ; | H 7.23 ; | Cl 23.13 ; | N 12.18 ; |
| % found : | C 47.13 ; | H 7.32 ; | Cl 22.92 ; | N 12.09 ; |

TLC: Support: silica gel plate (Merck G60)
Eluent: CHCl₃ : AcOH : H₂0 = 5 : 5 : 1
Developer : Cl₂ + o-Toluidine
Rf = 0.35
¹H-NMR, ¹³C-NMR and IR spectra agreed with the indicated structure.

### EXAMPLE 2

2-[1,4,7,10-tetraaza-7-(1-carboxy-2-benzyloxy-ethyl)-cyclododecane-1-yl]-3-benzyloxypropionic acid.

A solution of 12 g of 1,4,7,10-tetraazacyclododecane (0.069 mol) and 82.32 g of sodium 2-chloro-3-benzyloxypropionate (0.348 mol), obtained according to the process described in example 1-A, in 120 ml of DMF was placed in a sealed vessel and heated to 50°C for 30 h.

The salt formed was filtered and the solvent was distilled off under reduced pressure. The residue was taken up into 300 ml of water, the pH was adjusted to 2.5 with 10% hydrochloric acid, then the mixture was extracted with four 50 ml portions of methylene chloride.

The organic phase was evaporated to dryness and the residue was dissolved in 200 ml of 0.01N HCl and washed with ethyl ether.

The pH was adjusted to 6 with 10% sodium hydroxide and the aqueous solution was evaporated to dryness. The crude residue was taken up into 30 ml of water and adsorbed on amberlite IR 120, from which it was eluted with 5N ammonium hydroxide.

By concentration of the basic eluate, a residue of 7 g was obtained, which was crystallized from water.

5.85 g of 2-[1,4,7,10-tetraaza-7(1-carboxy-2-benzyloxyethyl)-cyclododecane-1-yl]-3-benzyloxypropionic acid (0.011 mol) were obtained.
Yield: 16% m.p.: 170°-175°C

| Elemental analysis: | | | |
|---|---|---|---|
| % calc: | C 63.61 ; | H 7.63 ; | N 10.60 |
| % found: | C 63.48 ; | H 7.82 ; | N 10.51 |

Analogously, the following compounds were obtained:
- 2-[1,4,7,10-tetraaza-4-(1-carboxy-2-benzyloxy-ethyl)-cyclododecane-1-yl]-3-benzyloxypropionic acid;
- 2-[1,4,7,10-tetraaza-4,7-di(1-carboxy-2-benzyloxy-ethyl)-cyclododecane-1-yl]-3-benzyloxypropionic acid.

### EXAMPLE 3

2-[1,4,7,10-tetraaza-4,7,10-tri(carboxymethyl)-cyclododecane-1-yl]-3-benzyloxypropionic acid. (Method A).

To a suspension of 23 g of 2-(1,4,7,10-tetraazacyclododecane-1-yl)-3-benzyloxypropionic acid trihydrochloride (0.05 mol), obtained according to the process described in example 1, and 27.8 g of bromoacetic acid (0.2 mol ) in 100 ml of water about 60 ml of 6N sodium hydroxide were added, under stirring, to reach pH=10. The mixture was heated to 50°C for 17 h and the pH was kept at 10 by further additions of 6N sodium hydroxide.

The solution was cooled and applied to amberlite IR 120, from which the product was eluted with 5N ammonium hydroxide. The basic eluate was evaporated to dryness, the resulting crude compound was dissolved in water and the solution was acidified to pH 3 with 5N HCl.

The precipitate was filtered and crystallized from water to give the desired compound.

15.3 g of 2-[1,4,7,10-tetraaza-4,7,10-tri(carboxymethyl)-cyclododecane-1-yl]-3-benzyloxypropionic acid (0.029 mol) were obtained.
Yield: 58.4% ; m.p.: 173°C with dec.
Titre:
(NaOH) : 99.6%
(ZnSO₄): 99.5%
(HPLC) : 99.0%

| Elemental analysis: | | | |
|---|---|---|---|
| % calc: | C 54.95 ; | H 6.92 ; | N 10.68 |
| % found: | C 54.77 ; | H 6.96 ; | N 10.77 |

¹H-NMR, ¹³C-NMR and IR spectra agreed with the indicated structure.

### EXAMPLE 4

2-[1,4,7,10-tetraaza-4,7,10-tri(carboxymethyl)-cyclododecane-1-yl]-3-benzyloxypropionic acid (Method B).

A mixture of 10 g of 2-(1,4,7,10-tetraazacyclododecane-1-yl)-3-benzyloxypropionic acid (0.028 mol), obtained according to the process described in example 1 but without formation of the hydrochloride, and 15.57 g of bromoacetic acid (0.112 mol) in 60 ml of water was treated according to the same process as in example 3, to give the desired compound.

7.93 g of 2-[1,4,7,10-tetraaza-4,7,10-tri(carboxymethyl)-cyclododecane-1-yl]-3-benzyloxypropionic acid (0.015 mol) were obtained.
Yield: 54% m.p.: 169-172°C with dec.
Titre:
(NaOH) : 99.3%
(ZnSO₄): 99.5%

| Elemental analysis: | | | |
|---|---|---|---|
| % calc.: | C 54.95 ; | H 6.92 ; | N 10.68 |
| % found: | C 54.71 ; | H 7.00 ; | N 10.64 |

The other chemico-physical characteristics agreed with the ones of the compound obtained according to method A (example 3).

Analogously, the following compounds were obtained:
- 2-[1,4,7,10-tetraaza-4,7,10-tri(1-carboxy-ethyl)-cyclododecane-1-yl]-3-benzyloxypropionic acid.
- 2-[1,4,7,10-tetraaza-4-(1-carboxy-2-benzyloxy-ethyl)-7,10 -di(carboxymethyl)-cyclododecane-1-yl]-3-benzyloxypropionic acid.
- 2-[1,4,7,10-tetraaza-7-(1-carboxy-2-benzyloxy-ethyl)-4,10 -di(carboxymethyl)-cyclododecane-1-yl]-3-benzyloxypropionic acid.
- 2-[1,4,7,10-tetraaza-4,7-di(1-carboxy-2-benzyloxy-ethyl)-10-carboxymethyl-cyclododecane-1-yl]-3-benzyloxypropionic acid.

### EXAMPLE 5

D(-)-N-methylglucamine salt of the Gd⁽³⁺⁾/2-[1,4,7,10-tetraaza-4,7,10-tri(carboxymethyl)-cyclododecane-1-yl]-3-benzyloxypropionic acid complex.

To a suspension of 100 g of 2-[1,4,7,10-tetraaza-4,7,10-tri(carboxymethyl)-cyclododecane-1-yl]-3-benzyloxypropionic acid (0.19 mol), obtained according to the process described in example 3, in 150 ml of water 36.6 g of D(-)-N-methylglucamine (0.187 mol) were added. 19.47 g of Gd₂O₃ (0.095 mol) were added to the solution and the resulting suspension was heated to 50°C for 4 hours. The reaction mixture was filtered and the pH was adjusted to 6.5 by means of a 10% aqueous D(-)-N-methylglucamine solution. The resulting solution was then evaporated and dried to give the desired compound.

159 g of D(-)-N-methylglucamine salt of the Gd⁽³⁺⁾/2-[1,4,7,10-tetraaza-4,7,10-tri(carboxymethyl)-cyclododecane-1-yl]-3-benzyloxypropionic acid complex (0.182 mol ) were obtained.
Yield: 95.8% m.p.: 137°C
Titre:
(HPLC) : 99.3%

| Elemental analysis: | | | | |
|---|---|---|---|---|
| % calc.: | C 42.56 ; | H 5.76 ; | Gd 17.99 ; | N 8.01 |
| % found: | C 42.42 ; | H 5.96 ; | Gd 17.63 ; | N 7.92 |

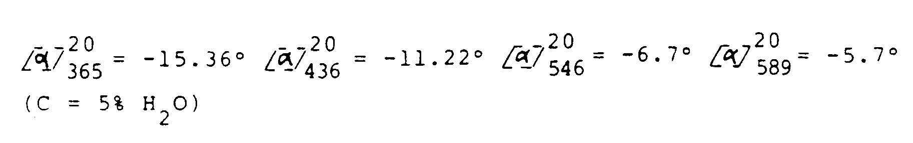

Analogously, the following compounds were obtained:
- Dy⁽³⁺⁾/2-[1,4,7,10-tetraaza-4,7,10-tri(carboxymethyl)-cyclododecane-1-yl]-3-benzyloxypropionic acid salt of D(-)-N-methylglucamine, obtained with Dy₂O₃.
- La⁽³⁺⁾/2-[1,4,7,10-tetraaza-4,7,10-tri(carboxymethyl)-cyclododecane-1-yl]-3-benzyloxypropionic acid salt of D(-)-N-methylglucamine, obtained with La₂O₃.
- Yb⁽³⁺⁾/2-[1,4,7,10-tetraaza-4,7,10-tri(carboxymethyl)-cyclododecane-1-yl]-3-benzyloxypropionic acid salt of D(-)-N-methylglucamine, obtained with Yb₂O₃.

### EXAMPLE 6

D(-)-N-methylglucamine salt of Gd⁽³⁺⁾/2-[1,4,7,10-tetraaza-4,7,10-tri(carboxymethyl)-cyclododecane-1-yl]-3-hydroxypropionic acid complex.

A solution of 92 g of D(-)-N-methylglucamine salt of Gd⁽³⁺⁾/2-[1,4,7,10-tetraaza-4,7,10-tri(carboxymethyl)-cyclododecane-1-yl]-3-benzyloxypropionic acid complex (0.105 mol), obtained according to the process described in example 5, in 550 ml of water, to which 153 g of 5% palladium on charcoal had been added, was hydrogenated for 5 h at room temperature.

The catalyst was removed by filtration and the aqueous solution was evaporated under vacuum at 50°C. Upon drying the residue to constant weight, the desired debenzylated compound was obtained.

67 g of D(-)-N-methylglucamine salt of Gd⁽³⁺⁾/2-[1,4,7,10-tetraaza-4,7,10-tri(carboxymethyl)-cyclododecane-1-yl]-3-hydroxypropionic acid complex (0.084 mol) were obtained.
Yield: 80% m.p.: 180°C (dec.)

| Elemental analysis: | | | | |
|---|---|---|---|---|
| % calc: | C 36.77 ; | H 5.65 ; | Gd 20.06 ; | N 8.93 |
| % found: | C 36.47 ; | H 5.47 ; | Gd 20.29 ; | N 8.83 |

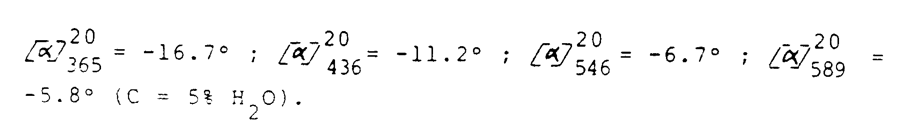

Analogously, the following compounds were obtained:
- 2-(1,4,7,10-tetraazacyclododecane-1-yl)-3-hydroxypropionic acid.
- 2-[1,4,7,10-tetraaza-4-(1-carboxy-2-hydroxy-ethyl)-cyclododecane-1-yl]-3-hydroxypropionic acid.
- 2-[1,4,7,10-tetraaza-7-(1-carboxy-2-hydroxy-ethyl)-cyclododecane-1-yl]-3-hydroxypropionic acid.
- 2-[1,4,7,10-tetraaza-4,7-di(1-carboxy-2-hydroxy-ethyl)-cyclododecane-1-yl]-3-hydroxypropionic acid.
- 2-[1,4,7,10-tetraaza-4-(1-carboxy-2-hydroxy-ethyl)-7,10-di(carboxymethyl)-cyclododecane-1-yl]-3-hydroxypropionic acid.
- 2-[1,4,7,10-tetraaza-7-(1-carboxy-2-hydroxy-ethyl)-4,10-di(carboxymethyl)-cyclododecane-1-yl]-3-hydroxypropionic acid.
- 2-[1,4,7,10-tetraaza-4,7-di(1-carboxy-2-hydroxy-ethyl)-10-carboxymethyl-cyclododecane-1-yl]-3-hydroxypropionic acid.
- 2-[1,4,7,10-tetraaza-4,7,10-tri(carboxymethyl)-cyclododecane-1-yl]-3-hydroxypropionic acid.

### EXAMPLE 7

2-(1,4,7,10-tetraazacyclododecane-1-yl)-3-benzyloxy-N-(1,3-dihydroxyisopropyl)-propionamide.
A) 3-benzyloxy-2-chloropropionylchloride.
119 g of thionyl chloride (1 mol) were added dropwise to 107.3 g of 3-benzyloxy-2-chloropropionic acid (0.5 mol) at 30°C. After refluxing the reaction mixture for 2 h, 33 g additional thionyl chloride (0.277 mol) were added and the mixture was refluxed for another 30 min.
Excess thionyl chloride was distilled off under reduced pressure and the desired compound was distilled under vacuum.
95.8 g of 3-benzyloxy-2-chloropropionylchloride (0.41 mol) were obtained.
Yield: 82% b.p.: 125-131°C; 0.05 mbar
Titre:
(reduction with Zn) : 99.9%
(Argentometric) : 96.0%

| Elemental analysis: | | | |
|---|---|---|---|
| % calc: | C 51.53% ; | H 4.32% ; | Cl 30.42% |
| % found: | C 51.30% ; | H 4.46% ; | Cl 29.48% |

¹H-NMR, ¹³C-NMR and IR spectra agreed with the indicated structure.
B) 2-Chloro-3-benzyloxy-N-(1,3-diyhdroxyisopropyl)-propionamide.
A solution of 70 g of 3-benzyloxy-2-chloropropionylchloride (0.3 mol) in 150 ml of tetrahydrofuran was added dropwise during about 2 h to a solution of 32.6 g of 2-amino-1,3-dihydroxyisopropane (0.36 mol) in 150 ml of water and 250 ml of tetrahydrofuran. During the addition of the chloride, the pH of the solution was kept constant at 10 by addition of 6N sodium hydroxide.
To the reaction mixture 250 ml of water were added. Upon concentration to 450 ml a white product precipitated, which was filtered and crystallized from water, after treatment with Carbopuron 4N to give the desired compound.
62.2 g of 2-chloro-3-benzyloxy-N-(1,3-dihydroxyisopropyl)-propionamide (0.218 mol) were obtained.
Yield: 72.6% m.p.: 133-135°C
Titre:
(Argentometric) : 99.8%

| Elemental analysis: | | | | | |
|---|---|---|---|---|---|
| % calc: | C 54.27 ; | H 6.30 ; | Cl 12.32 ; | N 4.87 | |
| % found: | C 54.19 ; | H 6.38 ; | Cl 12.24 ; | N 4.84 ; | H₂O 0.22 |

HPLC: 99%
¹H-NMR, ¹³C-NMR and IR spectra agreed with the indicated structure.
C) 2-(1,4,7,10-tetraazacyclododecane-1-yl)-3-benzyloxy-N-(1,3-dihydroxyisopropyl)-propionamide, trihydrochloride.
51.6 g of 1,4,7,10-tetraazacyclododecane (0.3 mol ) and 258.75 g of 2-chloro-3-benzyloxy-N-(1,3-dihydroxyisopropyl)-propionamide (0.9 mol), obtained according to the process described in example 7-B, were reacted at 70°C in DMF for 24 h.
After evaporation of the solvent under vacuum, the residue was taken up in water and adsorbed on an ion exchange resin IR 120, from which it was eluted by means of 5N ammonium hydroxide.
The ammonia solution was evaporated to dryness and the residue was transformed into the corresponding trihydrochloride, as described in example 1. 63.84 g of 2-(1,4,7,10-tetraazacyclododecane-1-yl)-3-benzyloxy-N-(1,3-dihydroxyisopropyl)-propionamide, trihydrochloride (0.120 mol) were obtained.
Yield: 40.0% m.p.: 125°C (dec.)

| Elemental analysis: | | | | |
|---|---|---|---|---|
| % calc: | C 47.33 ; | H 7.56 ; | Cl 19.96 ; | N 13.14 |
| % found: | C 47.41 ; | H 7.68 ; | Cl 19.85 ; | N 13.08 |

HPLC: 97.6%
Analogously, the following compounds were obtained:
- 2-(1,4,7,10-tetraazacyclododecane-1-yl)-3-benzyloxy-propionamide.
- 2-(1,4,7,10-tetraazacyclododecane-1-yl)-3-benzyloxy-N-(2-hydroxyethyl)-propionamide.
- 2-(1,4,7,10-tetraazacylododecane-1-yl)-3-benzyloxy-N-(2,3-dihydroxypropyl)-propionamide.
- 2-(1,4,7,10-tetraazacyclododecane-1-yl)-3-benzyloxy-N,N-di(2-hydroxyethyl)-propionamide.

### EXAMPLE 8

2-[1,4,7,10-tetraaza-4,7,10-tri(carboxymethyl)-cyclododecane-1-yl-]-3-benzyloxy-N-(1,3-dihydroxyisopropyl)-propionamide.

A mixture of 16 g of 2-(1,4,7,10-tetraazacyclododecane-1-yl)-3-benzyloxy-N-(1,3-dihydroxyisopropyl)-propionamide (0.038 mol), obtained according to the process described in example 7, and of 21.13 g of bromoacetic acid (0.152 mol) in 100 ml of water was reacted by the same process as described in example 3, to give the desired compound.

12.4 g of 2-[1,4,7,10-tetraaza-4,7,10-tri(carboxymethyl)-cyclododecane-1-yl]-3-benzyloxy-N-(1,3-dihydroxyisopropyl)-propionamide (0.0207 mol) were obtained.
Yield: 54.4% m.p.: 137°C (dec.)
Titre:
(NaOH) 98.8%

| Elemental analysis: | | | |
|---|---|---|---|
| % calc.: | C 53.98 ; | H 7.72 ; | N 11.66 |
| % found: | C 53.91 ; | H 7.85 ; | N 11.59 |

Analogously, the following compounds were obtained:
- 2-[1,4,7,10-tetraaza-4,7,10-tri(carboxymethyl)-cyclododecane-1-yl]-3-benzyloxy-propionamide.
- 2-[1,4,7,10-tetraaza-4,7,10-tri(carboxymethyl)-cyclododecane-1-yl]-3-benzyloxy-N-(2-hydroxyethyl)-propionamide.
- 2-[1,4,7,10-tetraaza-4,7,10-tri(carboxymethyl)-cyclododecane-1-yl]-3-benzyloxy-N-(2,3-dihydroxypropyl)-propionamide.
- 2-[1,4,7,10-tetraaza-4,7,10-tri(carboxymethyl)-cyclododecane-1-yl]-3-benzyloxy-N,N-di(2-hydroxyethyl)-propionamide.

### EXAMPLE 9

Gd⁽³⁺⁾/2-[1,4,7,10-tetraaza-4,7,10-tri(carboxymethyl)-cyclododecane-1-yl]-3-benzyloxy-N-(1,3-dihydroxyisopropyl)-propionamide.

To a suspension of 8 g of 2-[1,4,7,10-tetraaza-4,7,10-tri(carboxymethyl)-cyclododecane-1-yl]-3-benzyloxy-N-(1,3-dihydroxyisopropyl)-propionamide (0.013 mol), obtained according to the process described in example 8, in 30 ml of water 1.33 g of Gd₂O₃ (0.0065 mol) were added and the mixture was reacted at 50°C according to the procedure of example 5.

The resulting solution was evaporated to dryness to give the desired product.

9 g of Gd⁽³⁺⁾/2-[1,4,7,10-tetraaza-4,7,10-tri(carboxymethyl)-cyclododecane-1-yl]-3-benzyloxy-N-(1,3-dihydroxyisopropyl)-propionamide (0.012 mol) were obtained.
Yield: 92.3%

| Elemental analysis: | | | |
|---|---|---|---|
| % calc.: | C 42.95 ; | H 5.74 ; | N 9.28 |
| % found: | C 42.87 ; | H 5.80 ; | N 9.23 |

HPLC: 97.5%

### EXAMPLE 10

Gd⁽³⁺⁾/2-[1,4,7,10-tetraaza-4,7,10-tri(carboxymethyl)-cyclododecane-1-yl]-3-hydroxy-N-(1,3-dihydroxyisopropyl)-propionamide.

9 g of Gd⁽³⁺⁾/2-[1,4,7,10-tetraaza-4,7,10-tri(carboxymethyl)-cyclododecane-1-yl]-3-benzyloxy-N-(1,3-dihydroxyisopropyl)-propionamide (0.012 mol), obtained according to the process described in example 9, were dissolved in 60 ml of water. After addition of 15 g of 5% palladium on charcoal, the solution was hydrogenated according to the procedure of example 6 to give the desired compound.

6.22 g of Gd⁽³⁺⁾/2-[1,4,7,10-tetraaza-4,7,10-tri(carboxymethyl)-cyclododecane-1-yl]-3-hydroxy-N-(1,3-dihydroxyisopropyl)-propionamide (0.0096 mol) were obtained.
Yield: 80%

| Elemental analysis: | | | |
|---|---|---|---|
| % calc: | C 36.13 ; | H 5.61 ; | N 10.53 |
| % found: | C 36.06 ; | H 5.64 ; | N 10.48 |

Analogously, the following compounds were obtained:
- 2-(1,4,7,10-tetraazacyclododecane-1-yl)-3-hydroxy-propionamide
- 2-(1,4,7,10-tetraazacyclododecane-1-yl)-3-hydroxy-N-(2-hydroxyethyl)-propionamide.
- 2-(1,4,7,10-tetraazacyclododecane-1-yl)-3-hydroxy-N-(2,3-dihydroxypropyl)-propionamide.
- 2-(1,4,7,10-tetraazacyclododecane-1-yl)-3-hydroxy-N,N-di(2-hydroxyethyl)-propionamide.
- 2-[1,4,7,10-tetraaza-4,7,10-tri(carboxymethyl)-cyclododecane-1-yl]-3-hydroxy-propionamide.
- 2-[1,4,7,10-tetraaza-4,7,10-tri(carboxymethyl)-cyclododecane-1-yl]-3-hydroxy-N-(2-hydroxyethyl)-propionamide.
- 2-[1,4,7,10-tetraaza-4,7,10-tri(carboxymethyl)-cyclododecane-1-yl]-3-hydroxy-N-(2,3-dihydroxypropyl)-propionamide
   2-(1,4,7,10-tetraazacyclododecane-1-yl)-3-benzyloxy-N-(1,3-dihydroxyisopropyl)-propionamide,
   2-(1,4,7,10-tetraazacyclododecane-1-yl)-3-hydroxy-N-(1,3-dihydroxyisopropyl)-propionamide,
- 2-[1,4,7,10-tetraaza-4,7,10-tri(carboxymethyl)-cyclododecane-1-yl]-3-hydroxy-N,N-di(2-hydroxyethyl)-propionamide.

### EXAMPLE 11

Determination of the relaxivity of the compounds of the present invention.

| Operative conditions | |
|---|---|
| A) Apparatus: | MINISPEC PC 120 (BRUKER) |
| B) Observation frequency: | 20 MHz (proton) |
| C) Temperature: | 39°C, with pre-thermostatization of the NMR test tube for 10 min at the operative temperature |
| D) Concentrations: | in the range from 0 to 5 mM with the following specific measuring points: 0/0.1/0.2/0.5/1.0/2.0/5.0 mM |
| E) Solvent: | 0.154 M NaOH (0.9%), water |
| F) pH: | 7.3, to be checked again potentiometrically before the relaxation measurement. |

Longitudinal relaxivity (R₁) measurements were calculated using the "Inversion Recovery" sequence with an 8 point minimum and a 3 parameter fit, according to the program provided for the MINISPEC 126 BRUKER instrument, by which measurements were taken.

Transverse relaxivity (R₂) measurements were calculated using the sequence of Carr, Purcell, Meiboom and Gill, according to the program provided for the MINISPEC 120 BRUKER instrument, by means of which measurements were taken, adjusting the apparatus in such a way as to observe the decay of the signal to about 1/3 of the starting value, with a score number higher or equal to 10 and a 2 parameter fit.

In table I, R₁ and R ₂ values calculated for compounds A and B in comparison with Gd/DTPA neutralized with N-methylglucamine, are reported as non-limiting examples.

**TABLE I**

| | A (mM.s)⁻¹ | B (mM.s)⁻¹ | Gd/DTPA * (mM.s)⁻¹ |
|---|---|---|---|
| R₁ | 4.15±0.01 | 3.72±0.01 | 4.08±0.01 |
| R₂ | 5.67±0.02 | 5.06±0.01 | 5.15±0.02 |
| A = Gd⁽³⁺⁾/2-[1,4,7,10-tetraaza-4,7,10-tri(carboxymethyl)-cyclododecane-1-yl]-3-benzyloxypropionic acid, neutralized with N-methylglucamine. B = Gd⁽³⁺⁾/2-[1,4,7,10-tetraaza-4,7,10-tri(carboxymethyl)-cyclododecane-1-yl]-3-hydroxypropionic acid, neutralized with N-methylglucamine. | | | |

| | | | |
|---|---|---|---|
| * = neutralized with N-methylglucamine; R₁ and R₂ values were determined in aqueous solvent. | | | |

### EXAMPLE 12

Preparation of liposomes incorporating the Gd⁽³⁺⁾/2-[1,4,7,10-tetraaza-4,7,10-tri(carboxymethyl)-cyclododecane-1-yl]-3-benzyloxypropionic acid complex, neutralized with N-methylglucamine.

An anhydrous lipidic mixture was prepared, having the following composition: egg phosphatidylcholine 75 mol % and cholesterol 25 mol % using the REV method (F. Szoka et al., (1978), Proc. Natl. Acad. Sci. U.S.A. 75,4194).

400 mg of said mixture were dissolved in 35 ml of chloroform to which 10 ml of a 0.05 M solution of N-methyl-D-glucamine salt of Gd⁽³⁺⁾/2-[1,4,7,10-tetraaza-4,7,10-tri(carboxymethyl)-cyclododecane-1-yl]-3-benzyloxypropionic complex acid were added dropwise under sonication. When the addition was over, sonication was continued for 5 min, then the crude compound was heated to 50°C and the solvent was evaporated under vacuum. The resulting gelly residue was suspended in a 1% NaCl solution and freed from unincorporated chelate by means of five consecutive centrifugations and resuspension steps (26.000 g/10 min).

### EXAMPLE 13

Determination of LD₅₀ in the mouse by intravenous administration of the compounds of the present invention. In table II are reported, as non-limiting examples, the LD₅₀ values for compounds A and B of the present invention, in comparison with GdCl₃ and with Gd/DTPA neutralized with N-methylglucamine.

**TABLE II**

| | LD₅₀ in the mouse* in mmol/kg - intravenous |
|---|---|
| GdCl₃ | 0.28 (0.24 - 0.32) |
| Gd/DTPA** | 4.8 (4.47 - 5.16) |
| A | 8.8 (7.79 - 9.94) |
| B | 13.1 (12.2 - 14.1) |

| | |
|---|---|
| * = male and female mice were used, Strain: Crl:CDl(ICR)BR | |
| ** = N-methylglucamine salt. | |
| A = Gd⁽³⁺⁾/2-[1,4,7,10-tetraaza-4,7,10-tri(carboxymethyl)-cyclododecane-1-yl]-3-benzyloxypropionic acid, neutralized with N-methylglucamine. B = Gd⁽³⁺⁾/2-[1,4,7,10-tetraaza-4,7,10-tri(carboxymethyl)-cyclododecane-1-yl]-3-hydroxypropionic acid, neutralized with N-methylglucamine. | |

Table II shows that, in this pharmacological test, gadolinium complexes with the macrocyclic chelating agents of the invention have substantially decreased toxicities with respect to both GdCl₃ and Gd/DTPA.

### EXAMPLE 14

Preparation of a solution of D(-)-N-methylglucamine salt of Gd⁽³⁺⁾/ 2-[1,4,7,10-tetraaza-4,7,10-tri(carboxymethyl)-cyclododecane-1-yl]-3-benzyloxypropionic acid complex.

436.8 g (0.500 mol) of the compound obtained according to the procedure described in example 5 were dissolved in 300 ml of pro iniectione (p.i.) water. The solution volume was taken to 500 ml by addition of water p.i., then the solution was filtered, put in vials and sterilized.

### EXAMPLE 15

Preparation of a solution of D(-)-N-methylglucamine salt of Gd⁽³⁺⁾/ 2-[1,4,7,10-tetraaza-4,7,10-tri(carboxymethyl)-cyclododecane-1-yl]-3-hydroxypropionic acid complex.

398.8 g (0.500 mol) of the compound obtained according to the procedure described in example 6, were dissolved in 300 ml of water p.i.. The solution volume was taken to 500 ml by addition of water p.i., then the solution was filtered, put in vials and sterilized.

### EXAMPLE 16

Preparation of a solution of D(-)-N-methylglucamine salt of Gd⁽³⁺⁾/2-[1,4,7,10-tetraaza-4,7,10-tri(carboxymethyl)-cyclododecane -1-yl]-3-benzyloxypropionic acid complex.

218.4 g (0.250 mol) of the salt cited in example 14 were dissolved in 260 ml of water p.i., 0.6 g of ascorbic acid were added and the solution was diluted to 500 ml with water p.i. The solution was sterilized by filtration and put in vials.

### EXAMPLE 17

Preparation of a solution of the D(-)-N-methylglucamine salt of Gd⁽³⁺⁾ / 2-[1,4,7,10-tetraaza-4,7,10-tri(carboxymethyl)-cyclododecane-1-yl]-3-benzyloxypropionic acid complex.

218.4 g (0.250 mol) of the salt cited in example 14 were dissolved in 200 ml of water p.i., 0.45 g of tromethamine hydrochloride were added and the solution was diluted to 500 ml with water p.i. The solution was filtered, put in vials and sterilized.

### EXAMPLE 18

Preparation of a solution of the D(-)-N-methylglucamine salt of Gd⁽³⁺⁾/ 2-[1,4,7,10-tetraaza-4,7,10-tri(carboxymethyl)-cyclododecane-1-yl]-3-hydroxypropionic acid complex.

199.4 g (0.250 mol) of the salt cited in example 15 were dissolved in 200 ml of water p.i., 0.6 g of ascorbic acid were added and the solution was diluted to 500 ml with water p.i. The solution was sterilized by filtration and put into vials.

### EXAMPLE 19

Preparation of a solution of the D(-)-N-methylglucamine salt of Gd⁽³⁺⁾/ 2-[1,4,7,10-tetraaza-4,7,10-tri(carboxymethyl)-cyclododecane-1-yl]-3-hydroxypropionic acid complex.

199.4 g (0.250 mol) of the salt cited in example 15 were dissolved in 200 ml of water p.i., 0.45 g of tromethamine hydrochloride were added and the solution was diluted to 500 ml with water p.i. The solution was filtered, put into vials and sterilized.

## Claims (Claims for the following Contracting State(s): AT, BE, CH, DE, FR, GB, IT, LI, LU, NL, SE)

1. 1,4,7,10-tetraazacyclododecane of general formula I 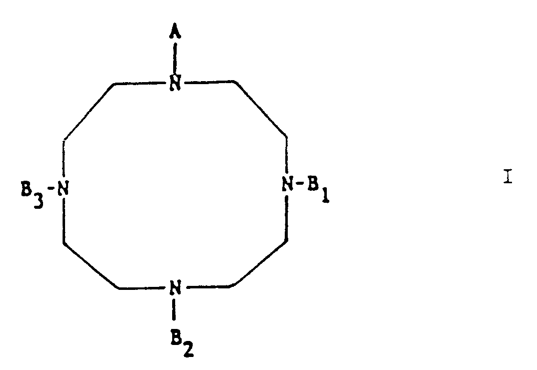 wherein
A is a group of formula 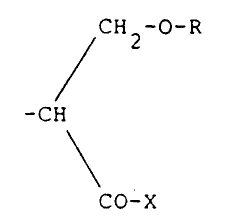
in which
R is H or a C₁-C₅ straight or branched alkyl group, or a benzyl group which can be mono- or poly-substituted on the aromatic ring by halogen, hydroxy, carboxy, carbamoyl, alkoxycarbonyl, sulphamoyl, lower alkyl, lower hydroxyalkyl, amino, acylamino, acyl, hydroxyacyl groups, or a group of formula H(OCH₂CH₂)₁₋₄-, Me(OCH₂CH₂)₁₋₄-, or Et(OCH₂CH₂)₁₋₄-,
X is a O-R₁ group in which R₁ is H or a C₁-C₅ alkyl, hydroxyalkyl, alkoxyalkyl, alkoxyhydroxyalkyl group, or a polyoxaalkyl group having 1 to 15 oxygen atoms and 3 to 45 carbon atoms, or X is a -NR₂R₃ group in which R₂ and R₃, which can be the same or different, are hydrogen, C₁-C₆ alkyl, hydroxyalkyl, alkoxyalkyl or alkoxyhydroxyalkyl groups having up to 5 hydroxy groups
and
B₁, B₂ and B₃, which can be the same or different, have the same meaning as A or they are H or a group of formula 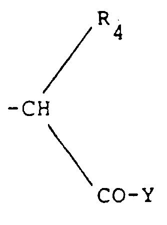 in which
R₄ is H or a C₁-C₅ straight or branched alkyl group,
Y is a O-R₅ group in which R₅ is H or a C₁-C₅ alkyl, hydroxyalkyl, alkoxyalkyl, alkoxyhydroxyalkyl group, or a polyoxaalkyl group having 1 to 15 oxygen atoms and 3 to 45 carbon atoms, or Y is a -NR₆R₇ group in which R₆ and R₇, which can be the same or different, are H or C₁-C₆ alkyl, hydroxyalkyl, alkoxyalkyl or alkoxyhydroxyalkyl groups having up to 5 hydroxy groups,
said derivatives being, if necessary, salified with suitable organic or inorganic bases,
as well as the chelates of compound of formula I with metal ions, which chelates, if necessary, can in turn be neutralized with inorganic or organic acid or bases.

2. 1,4,7,10-Tetraazacyclododecane derivatives of general formula II 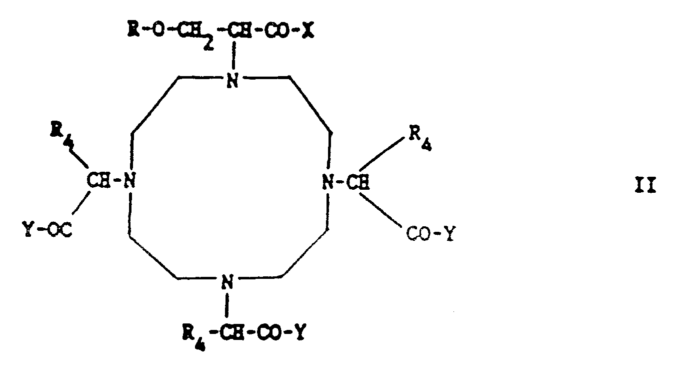 wherein
R,R₄,X and Y have the meanings defined in claim 1, and the chelates thereof with bi- or trivalent ions of metal elements having atomic numbers from 20 to 31, 39, 42, 43, 44, 49 or from 57 to 83.

3. 1,4,7,10-Tetraazacyclododecane derivatives of general formula III 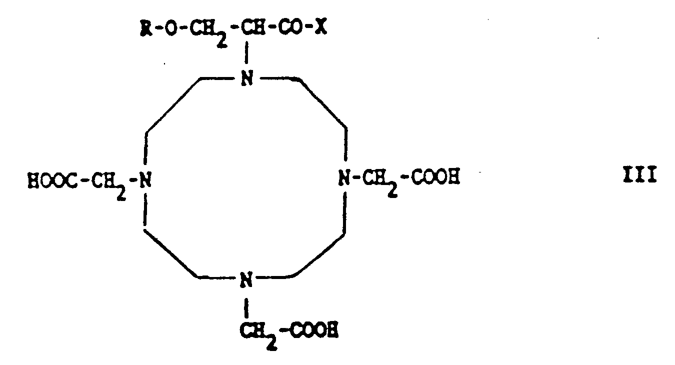 wherein R and X have the meanings defined in claim 1, and the chelates thereof with bi- or trivalent ions of metal elements having atomic numbers from 20 to 31, 39, 42, 43, 44, 49 or from 57 to 83.

4. Serine derivatives of general formula IV 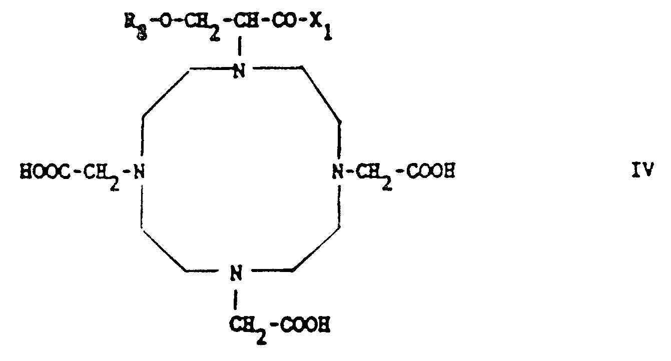 wherein R₈ is H or benzyl, X₁ is OH, -NH₂, - NHCH₂CH₂OH, -NHCH(CH₂OH)₂, -NHCH₂CH(OH)CH₂OH, -N(CH₂CH₂OH)₂, -NH-CH₂-CH(OH)-CH₂OCH₃ or -NH-CH₂-CH(OH)-CH(OH)-CH₂OH, and the chelates thereof with bi- or trivalent ions of metal elements having atomic numbers from 20 to 31, 38, 42, 43, 44, 49, or from 57 to 83.

5. A chelate of a compound as claimed in claims 1-4, in which the chelate metal ion is Fe⁽²⁺⁾, Fe⁽³⁺⁾, Cu⁽²⁺⁾, Gd⁽³⁺⁾, Eu⁽³⁺⁾, Dy⁽³⁺⁾ or Mn⁽²⁺⁾.

6. Chelates as claimed in claim 2 with the ions of the following radioisotopes: ⁵¹Cr, ⁶⁸Ga, ¹¹¹In, ^{99m}Tc, ¹⁴⁰La, ¹⁶⁸Yb.

7. A compound as claimed in claims 1-6, selected from the group consisting of:
- 2-(1,4,7,10-tetraazacyclododecane-1-yl)-3-benzyloxypropionic acid,
- 2-(1,4,7,10-tetraazacyclododecane-1-yl)-3-hydroxypropionic acid,
- 2-[1,4,7,10-tetraaza-4-(1-carboxy-2-benzyloxy-ethyl)-cyclododecane-1-yl]-3-benzyloxypropionic acid,
- 2-[1,4,7,10-tetraaza-4-(1-carboxy-2-hydroxy-ethyl)-cyclododecane-1-yl]-3-hydroxypropionic acid,
- 2-[1,4,7,10-tetraaza-7-(1-carboxy-2-benzyloxy-ethyl)-cyclododecane-1-yl]-3-benzyloxypropionic acid,
- 2-[1,4,7,10-tetraaza-7-(1-carboxy-2-hydroxy-ethyl)-cyclododecane-1-yl]-3-hydroxypropionic acid,
- 2-[1,4,7,10-tetraaza-4,7-di(1-carboxy-2-benzyloxy-ethyl)-cyclododecane-1-yl]-3-benzyloxypropionic acid,
- 2-[1,4,7,10-tetraaza-4,7-di(1-carboxy-2-hydroxy-ethyl)-cyclododecane-1-yl]-3-hydroxypropionic acid,
- 2-[1,4,7,10-tetraaza-4-(1-carboxy-2-benzyloxy-ethyl)-7,10-di(carboxymethyl)-cyclododecane-1-yl]-3-benzyloxypropionic acid,
- 2-[1,4,7,10-tetraaza-4-(1-carboxy-2-hydroxy-ethyl)-7,10-di(carboxymethyl)-cyclododecane-1-yl]-3-hydroxypropionic acid,
- 2-[1,4,7,10-tetraaza-7-(1-carboxy-2-benzyloxy-ethyl)-4,10-di(carboxymethyl)-cyclododecane-1-yl]-3-benzyloxypropionic acid,
- 2-[1,4,7,10-tetraaza-7-(1-carboxy-2-hydroxy-ethyl)-4,10-di(carboxymethyl)-cyclododecane-1-yl]-3-hydroxypropionic acid,
- 2-[1,4,7,10-tetraaza-4,7-di(1-carboxy-2-benzyloxy-ethyl)-10-carboxymethyl-cyclododecane-1-yl]-3-benzyloxypropionic acid,
- 2-[1,4,7,10-tetraaza-4,7-di(1-carboxy-2-hydroxy-ethyl)-10-carboxymethyl-cyclododecane-1-yl]-3-hydroxypropionic acid,
- 2-[1,4,7,10-tetraaza-4,7,10-tri(carboxymethyl)-cyclododecane-1-yl]-3-benzyloxypropionic acid,
- 2-[1,4,7,10-tetraaza-4,7,10-tri(carboxymethyl)-cyclododecane-1-yl]-3-hydroxypropionic acid,
- 2-(1,4,7,10-tetraazacyclododecane-1-yl)-3-benzyloxy-propionamide,
- 2-(1,4,7,10-tetraazacyclododecane-1-yl)-3-hydroxy-propionamide,
- 2-(1,4,7,10-tetraazacyclododecane-1-yl)-3-benzyloxy-N-(2-hydroxyethyl)-propionamide,
- 2-(1,4,7,10-tetraazacyclododecane-1-yl)-3-hydroxy-N-(2-hydroxyethyl)-propionamide,
- 2-(1,4,7,10-tetraazacyclododecane-1-yl)-3-benzyloxy-N-(1,3-dihydroxyisopropyl)-propionamide,
- 2-(1,4,7,10-tetraazacyclododecane-1-yl)-3-hydroxy-N-(1,3-dihydroxyisopropyl)-propionamide,
- 2-(1,4,7,10-tetraazacyclododecane-1-yl)-3-benzyloxy-N-(2,3-dihydroxypropyl)-propionamide,
- 2-(1,4,7,10-tetraazacyclododecane-1-yl)-3-hydroxy-N-(2,3-dihydroxypropyl)-propionamide,
- 2-(1,4,7,10-tetraazacyclododecane-1-yl)-3-benzyloxy-N,N-di(2-hydroxyethyl)-propionamide,
- 2-(1,4,7,10-tetraazacyclododecane-1-yl)-3-hydroxy-N,N-di(2-hydroxyethyl)-propionamide,
- 2-[1,4,7,10-tetraaza-4,7,10-tri(carboxymethyl)-cyclododecane-1-yl]-3-benzyloxy-propionamide,
- 2-[1,4,7,10-tetraaza-4,7,10-tri(carboxymethyl)-cyclododecane-1-yl]-3-hydroxy-propionamide,
- 2-[1,4,7,10-tetraaza-4,7,10-tri(carboxymethyl)-cyclododecane-1-yl]-3-benzyloxy-N-(2-hydroxyethyl)-propionamide,
- 2-[1,4,7,10-tetraaza-4,7,10-tri(carboxymethyl)-cyclododecane-1-yl]-3-hydroxy-N-(2-hydroxyethyl)-propionamide,
- 2-[1,4,7,10-tetraaza-4,7,10-tri(carboxymethyl)-cyclododecane-1-yl]-3-benzyloxy-N-(1,3-dihydroxyisopropyl)-propionamide,
- 2-[1,4,7,10-tetraaza-4,7,10-tri(carboxymethyl)-cyclododecane-1-yl]-3-hydroxy-N-(1,3-dihydroxyisopropyl)-propionamide,
- 2-[1,4,7,10-tetraaza-4,7,10-tri(carboxymethyl)-cyclododecane-1-yl]-3-benzyloxy-N-(2,3-dihydroxypropyl)-propionamide,
- 2-[1,4,7,10-tetraza-4,7,10-tri(carboxymethyl)-cyclododecane-1-yl]-3-hydroxy-N-(2,3-dihydroxypropyl)-propionamide,
- 2-[1,4,7,10-tetraaza-4,7,10-tri(carboxymethyl)-cyclododecane-1-yl]-3-benzyloxy-N,N-di(2-hydroxyethyl)-propionamide,
- 2-[1,4,7,10-tetraaza-4,7,10-tri(carboxymethyl)-cyclododecane-1-yl]-3-hydroxy-N,N-di(2-hydroxyethyl)-propionamide,
the respective chelate complexes with Fe⁽³⁺⁾, Cu⁽²⁺⁾, Mn⁽²⁺⁾, Gd⁽³⁺⁾, Dy⁽³⁺⁾, In⁽³⁺⁾, La⁽³⁺⁾, Yb⁽³⁺⁾ and the corresponding salts with D(-)-N-methylglucamine.

8. A method for the preparation of metal chelates of compounds of formula I of claim 1, which consists in reacting a salt or a metal oxide selected from the ones having atomic numbers from 20 to 31, 39, 42, 43, 44, 49 or from 57 to 83 with a compound of formula I of claim 1, possibly in the presence of the acid or base amount necessary for neutralization.

9. A diagnostic Preparation comprising a) a physiologically acceptable complex salt, in which the chelating agent is a compound of general formula I according to claimes 1-4 and the metal ion is selected from the group of the elements having atomic numbers comprised from 20 to 31, 38, from 42 to 44, 49 or from 57 to 83, b) if necessary, an ion of an inorganic or organic acid or base, c) a suitable physiologically acceptable carrier, d) eventually, physiologically acceptable additives.

10. A diagnostic preparation according to claim 9, in which the complex salt is present in a concentration from 0,002 to 1 mol/l.

## Claims (Claims for the following Contracting State(s): ES)

1. A process for the preparation of 1,4,7,10-tetraazacyclododecane of general formula I 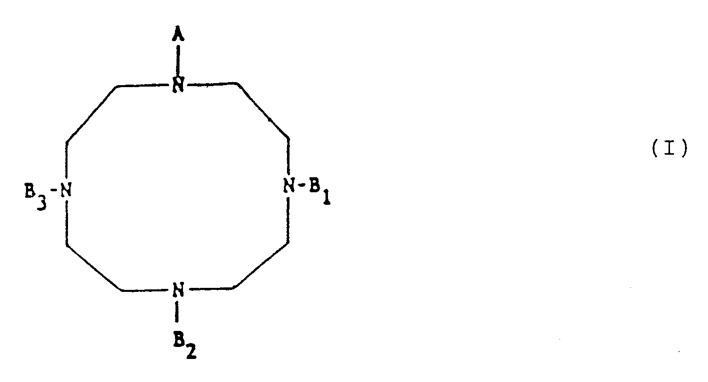 wherein
A is a group of formula 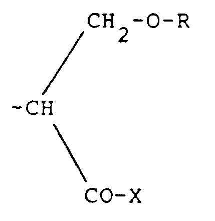 in which
R is h or a C₁-C₅ straight or branched alkyl group, or a benzyl group which can be mono- or poly-substituted on the aromatic ring by halogen, hydroxy, carboxy, carbamoyl, alkoxycarbonyl, sulphamoyl, lower alkyl, lower hydroxyalkyl, amino, acylamino, acyl, hydroxyacyl groups, or a group of formula H(OCH₂CH₂)₁₋₄-, Me(OCH₂CH₂)₁₋₄-, or Et(OCH₂CH₂)₁₋₄-,
X is a O-R₁ group in which R₁ is H or a C₁-C₅ alkyl, hydroxyalkyl, alkoxyalkyl, alkoxyhydroxyalkyl group, or a polyoxaalkyl group having 1 to 15 oxygen atoms and 3 to 45 carbon atoms, or X is a -NR₂R₃ group in which R₂ and R₃, which can be the same or different, are hydrogen, C₁-C₆ alkyl, hydroxyalkyl, alkoxyalkyl or alkoxyhydroxyalkyl group having up to 5 hydroxy groups
and
B₁, B₂ e B₃, which can be the same or different, have the same meaning as A or they are H or a group of formula 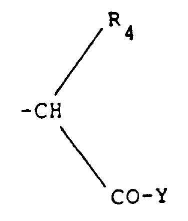 in which
R₄ is H or a C₁-C₅ straight or branched alkyl group,
Y is a O-R₅ group in which R₅ is H or a C₁-C₅ alkyl, hydroxyalkyl, alkoxyalkyl, alkoxyhydroxyalkyl group, or a polyoxaalkyl group having 1 to 15 oxygen atoms and 3 to 45 carbon atoms, or Y is a -NR₆R₇ group in which R₆ and R₇, which can be the same or different, are H or , C₁-C₆ alkyl, hydroxyalkyl, alkoxyalkyl or alkoxyhydroxyalkyl group having up to 5 hydroxy groups,
characterized by the following steps:
a) reacting 1,4,7,10-tetraazacyclododecane (II) 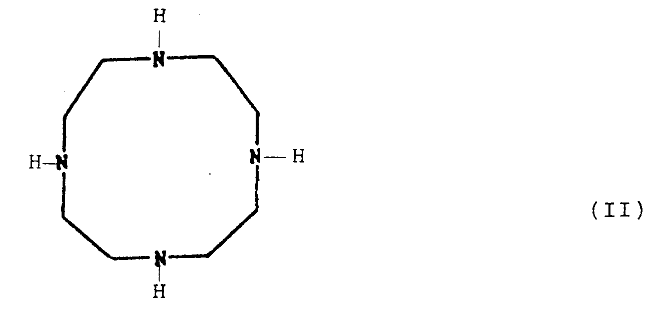 with an α-halo-propionyl derivative (III) 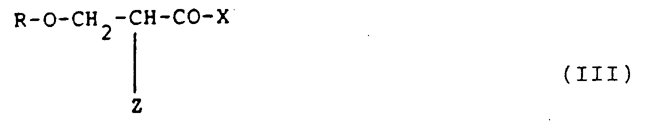 wherein Z is halogen and R and X have the above defined meanings, to give the addition product IV 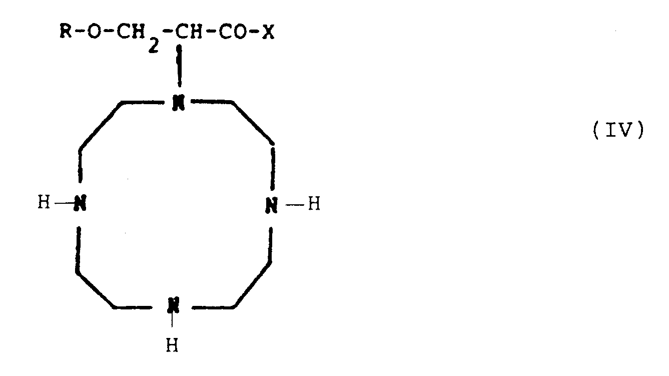 or the corresponding polysubstituted products on the nitrogen atoms of II, depending on the excess of III used;
b) condensing compound (IV) or the polysubstituted analogues thereof with the appropriate α-halo-acetic derivative (VIII), or a suitable precursor thereof 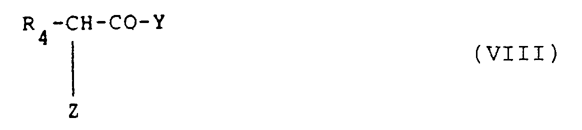 wherein Z is halogen and R₄ and Y have the above defined meanings, to give the desired chelating agents of general formula I.

2. A process according to claim 1, characterized by the following steps:
a) reacting a diethylenetriamine (V),
H2NCH2CH2NHCH2CH2NH2 (V)
which has previously been protected with a suitable protesting group, with the halo-derivative (III) to give compound (VII) 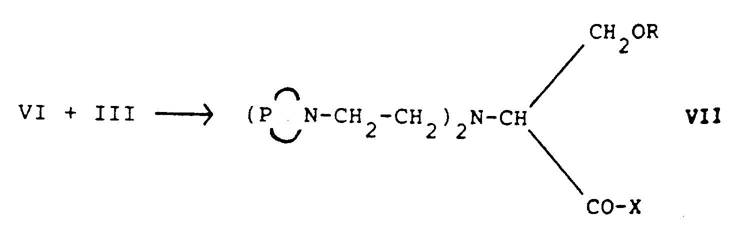 wherein P is said protecting group, R and X are as above defined;
b) eliminating group P from compound (VII);
c) condensing deprotected compound (VII) with tosyldiethanolamine, to give compound (IV);
d) condensing compound (IV) with the appropriate - halo-acetic derivative (VIII) or a suitable precursor thereof.

3. A process for the preparation of metal chelates of compounds of formula I of claim 1, which consists in reacting a salt or a metal oxide selected from the ones having atomic numbers from 20 to 31, 39, 42, 43, 44, 49 or from 57 to 83 with a compound of formula I of claim 1, possibly in the presence of the acid or base amount necessary for neutralization.

## Patentansprüche (Patentansprüche für folgende(n) Vertragsstaat(en): AT, BE, CH, DE, FR, GB, IT, LI, LU, NL, SE)

1. 1,4,7,10-Tetraazacyclododecan der allgemeinen Formel I 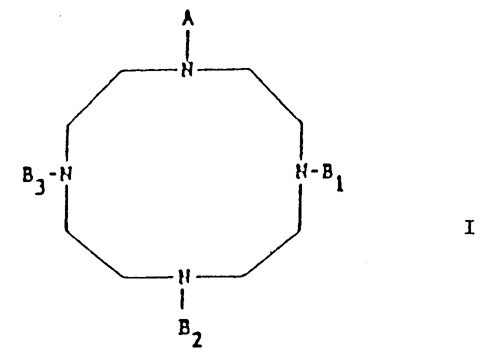 worin
A = eine Gruppe der Formel 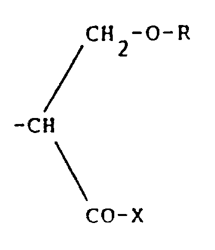
bei welcher
R = H oder eine C₁-C₅ gerade oder verzweigte Alkyl-Gruppe, oder eine Benzyl-Gruppe, die bei dem aromatischen Ring mono- oder substituiert werden kann durch Halogen-, Hydroxy-, Carboxy-, Carbamoyl-, Alkoxycarbonyl-, Sulphamoyl-, niedrige Alkyl-, niedrige Hydroxyalkyl-, Amino-, Acylamino-, Acyl-, Hydroxyacyl-Gruppen, oder eine Gruppe der Formel H(OCH₂CH₂)₁₋₄-, Me(OCH₂CH₂)₁₋₄-, oder Et(OCH₂CH₂)₁₋₄-,
X = eine O-R₁-Gruppe, bei welcher R₁ = H oder eine C₁-C₅ Alkyl-, Hydroxyalkyl-, Alkoxyalkyl-, Alkoxyhydroxyalkyl-Gruppe, oder eine Polyoxaalyl-Gruppe mit 1 bis 15 Sauerstoff-Atomen und 3 bis 45 Kohlenstoffatomen, oder X = eine -NR₂R₃-Gruppe, bei welcher R₂ und R₃, die gleich oder verschieden sein können, Wasserstoff, C₁-C₆ Alkyl-, Hydroxyalkyl-, Alkoxyalkyl- oder Alkoxyhydroxyalkyl-Gruppen mit bis zu 5 Hydroxy-Gruppen sind
und
B₁, B₂ und B₃, die gleich oder verschieden sein können, dieselbe Bedeutung haben wie A oder H oder eine Gruppe sind mit der Formel 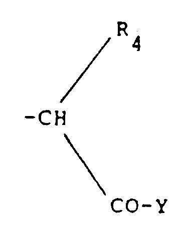 bei welcher
R₄ = H oder eine C₁-C₅ gerade oder verzweigte Alkyl-Gruppe,
Y = eine O-R₅-Gruppe, bei welcher R₅ = H oder eine C₁-C₅ Alkyl-, Hydroxyalkyl-, Alkoxyalkyl- oder Alkoxyhydroxyalkyl-Gruppe, oder eine Polyoxaalkyl-Gruppe mit 1 bis 15 Sauerstoffatomen und 3 bis 45 Kohlenstoffatomen, oder Y = eine -NR₆R₇-Gruppe, bei welcher R₆ und R₇, die gleich oder verschieden sein können, H oder C₁-C₆ Alkyl, Hydroxyalkyl-, Alkoxyalkyl- oder Alkoxyhydroxyalkyl-Gruppen sind mit bis zu 5 Hydroxy-Gruppen,
die Derivate, falls erforderlich, mit geeigneten organischen oder anorganischen Basen versalzt sind, sowie die Chelate der Verbindung der Formel I, mit Metallionen, wobei die Chelate ihrerseits, falls nötig, mit anorganischer oder organischer Säure oder Basen neutralisiert sein können.

2. 1,4,7,10-Tetraazacyclododecan-Derivate der allgemeinen Formel II 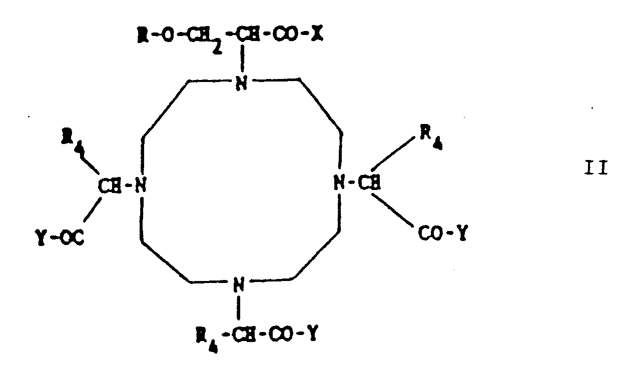 worin
R,R₄,X und Y die in Anspruch 1 definierten Bedeutungen haben, und die Chelate davon mit bi- oder trivalenten Ionen von Metallelementen die Ordnungszahlen von 20 bis 31, 39, 42, 43, 44, 49 oder von 57 bis 83 haben.

3. 1,4,7,10-Tetraazacyclododecan-Derivate der allgemeinen Formel III 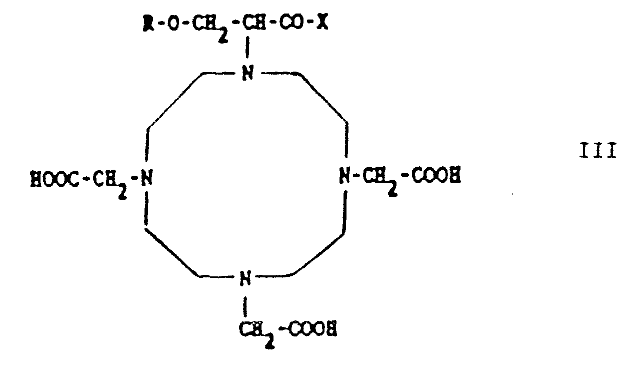 worin R und X die in Anspruch 1 definierten Bedeutungen haben, und die Chelate davon mit bi- oder trivalenten Ionen von Metallelementen die Ordnungszahlen von 20 bis 31, 39, 42, 43, 44, 49 oder von 57 bis 83 haben.

4. Serin-Derivat der allgemeinen Formel IV 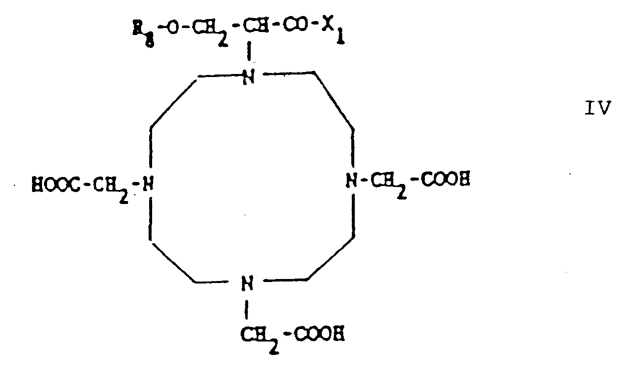 worin R₈ = H oder Benzyl, X₁ = OH, -NH₂, - NHCH₂CH₂OH, -NHCH(CH₂OH)₂, -NHCH₂CH(OH)CH₂OH, -N(CH₂CH₂OH)₂, -NH-CH₂-CH(OH)-CH₂OCH₃ oder -NH-CH₂-CH(OH)-CH(OH)-CH₂OH, und die Chelate davon mit mit bi- oder trivalenten Ionen von Metallelementen die Ordnungszahlen von 20 bis 31, 39, 42, 43, 44, 49 oder von 57 bis 83 haben.

5. Chelat einer Verbindung nach den Ansprüchen 1 bis 4, bei welcher das Chelat-Metallinon Fe⁽²⁺⁾, Fe⁽³⁺⁾, Cu⁽²⁺⁾, Gd⁽³⁺⁾, Eu⁽³⁺⁾, Dy⁽³⁺⁾ oder Mn⁽²⁺⁾ ist.

6. Chelate nach Anspruch 2 mit den Ionen der folgenden Radioisotope: ⁵¹Cr, ⁶⁸Ga, ¹¹¹In, ^{99m}Tc, ¹⁴⁰La, ¹⁶⁸Yb.

7. Verbindung nach den Ansprüchen 1 bis 6, ausgewählt aus der Gruppe bestehend aus:
- 2-(1,4,7,10-Tetraazacyclododecan-1-yl)-3-Benzyloxypropionsäure,
- 2-(1,4,7,10-Tetraazacyclododecan-1-yl)-3-Hydroxypropionsäure,
- 2-[1,4,7,10-Tetraaza-4-(1-Carboxy-2-Benzyloxy-Ethyl)-Cyclododecan-1-yl]-3-Benzyloxypropionsäure,
- 2-[1,4,7,10-Tetraaza-4-(1-Carboxy-2-Hydroxy-Ethyl)-Cyclododecan-1-yl]-3-Hydroxypropionsäure,
- 2-[1,4,7,10-Tetraaza-7-(1-Carboxy-2-Benzyloxy-Ethyl)-Cyclododecan-1-yl]-3-Benzyloxypropionsäure,
- 2-[1,4,7,10-Tetraaza-7-(1-Carboxy-2-Hydroxy-Ethyl)-Cyclododecan-1-yl]-3-Hydroxypropionsäure,
- 2-[1,4,7,10-Tetraaza-4,7-di(1-Carboxy-2-Benzyloxy-Ethyl)-Cyclododecan-1-yl]-3-Benzyloxypropionsäure,
- 2-[1,4,7,10-Tetraaza-4,7-di(1-Carboxy-2-Hydroxy-Ethyl)-Cyclododecan-1-yl]-3-Hydroxypropionsäure,
- 2-[1,4,7,10-Tetraaza-4-(1-Carboxy-2-Benzyloxy-Ethyl)-7,10-di(Carboxymethyl)-Cyclododecan-1-yl]-3-Benzyloxypropionsäure,
- 2-[1,4,7,10-Tetraaza-4-(1-Carboxy-2-Hydroxy-Ethyl)-7,10-di (Carboxymethyl)-Cyclododecan-1-yl]-3-Hydroxypropionsäure,
- 2-[1,4,7,10-Tetraaza-7-(1-Carboxy-2-Benzyloxy-Ethyl)-4,10-di(Carboxymethyl)-Cyclododecan-1-yl]-3-Benzyloxypropionsäure,
- 2-[1,4,7,10-Tetraaza-7-(1-Carboxy-2-Hydroxy-Ethyl)-4,10-di(Carboxymethyl)-Cyclododecan-1-yl]-3-Hydroxypropionsäure,
- 2-[1,4,7,10-Tetraaza-4,7-di(1-Carboxy-2-Benzyloxy-Ethyl)-10-Carboxymethyl-Cyclododecan-1-yl]-3-Benxyloxypropionsäure,
- 2-[1,4,7,10-Tetraaza-4,7-di(1-Carboxy-2-Hydroxy-Ethyl)-10-Carboxymethyl)-Cyclododecan-1-yl]-3-Hydroxypropionsäure,
- 2-[1,4,7,10-Tetraaza-4,7,10-tri(Carboxymethyl)-Cyclododecan -1-yl]-3-Benzyloxypropionsäure,
- 2-[1,4,7,10-Tetraaza-4,7,10-tri(Carboxymethyl)-Cyclododecan -1-yl]-3-Hydroxypropionsäure,
- 2-(1,4,7,10-Tetraazacyclododecan-1-yl)-3-Benzyloxy-Propionamid,
- 2-(1,4,7,10-Tetraazacyclododecan-1-yl)-3-Hydroxy-Propionamid,
- 2-(1,4,7,10-Tetraazacyclododecan-1-yl)-3-Benzyloxy-N-(2-Hydroxyethyl)-Propionamid,
- 2-(1,4,7,10-Tetraazacyclododecan-1-yl)-3-Hydroxy-N-(2-Hydroxyethyl)-Propionamid,
- 2-(1,4,7,10-Tetraazacyclododecan-1-yl)-3-Benzyloxy-N-(1,3-Dihydroxyisopropyl)-Propionamid,
- 2-(1,4,7,10-Tetraazacyclododecan-1-yl)-3-Hydroxy-N-(1,3-Dihydroxyisopropyl)-Propionamid,
- 2-(1,4,7,10-Tetraazacyclododecan-1-yl)-3-Benzyloxy-N-(2,3-Dihydroxypropyl)-Propionamid,
- 2-(1,4,7,10-Tetraazacyclododecan-1-yl)-3-Hydroxy-N-(2,3-Dihydroxypropyl)-Propionamid,
- 2-(1,4,7,10-Tetraazacyclododecan-1-yl)-3-Benzyloxy-N-N-di(2-Hydroxyethyl)-Propionamid,
- 2-(1,4,7,10-Tetraazacyclododecan-1-yl)-3-Hydroxy-N,N-di(2-Hydroxyethyl)-Propionamid,
- 2-[1,4,7,10-Tetraaza-4,7,10-tri(Carboxymethyl)-Cyclododecan-1-yl]-3-Benzyloxy-Propionamid,
- 2-[1,4,7,10-Tetraaza-4,7,10-tri(Carboxymethyl)-Cyclododecan-1-yl]-3-Hydroxy-Propionamid,
- 2-[1,4,7,10-Tetraaza-4,7,10-tri(Carboxymethyl)-Cyclododecan-1-yl]-3-Benzyloxy-N-(2-Hydroxyethyl)-Propionamid,
- 2-[1,4,7,10-Tetraaza-4,7,10-tri(Carboxymethyl)-Cyclododecan-1-yl]-3-Hydroxy-N-(2-Hydroxyethyl)-Propionamid,
- 2-[1,4,7,10-Tetraaza-4,7,10-tri(Carboxymethyl)-Cyclododecan-1-yl]-3-Benzyloxy-N-(1,3-Dihydroxyisopropyl)-Propionamid,
- 2-[1,4,7,10-Tetraaza-4,7,10-tri(Carboxymethyl)-Cyclododecan-1-yl]-3-Hydroxy-N-(1,3-Dihydroxyisopropyl)-Propionamid,
- 2-[1,4,7,10-Tetraaza-4,7,10-tri(Carboxymethyl)-Cyclododecan-1-yl]-3-Benzyloxy-N-(2,3-Dihydroxypropyl)-Propionamid,
- 2-[1,4,7,10-Tetraaza-4,7,10-tri(Carboxymethyl)-Cyclododecan-1-yl]-3-Hydroxy-N-(2,3-Dihydroxypropyl)-Propionamid,
- 2-[1,4,7,10-Tetraaza-4,7,10-tri(Carboxymethyl)-Cyclododecan-1-yl]-3-Benzyloxy-N,N-di(2-Hydroxyethyl)-Propionamid,
- 2-[1,4,7,10-Tetraaza-4,7,10-tri(Carboxymethyl)-Cyclododecan-1-yl]-3-Hydroxy-N-N-di(2-Hydroxyethyl)-Propionamid,
die betreffenden Chelatkomplexe mit Fe⁽³⁺⁾, Cu⁽²⁺⁾, Mn⁽²⁺⁾, Gd⁽³⁺⁾, Dy⁽³⁺⁾, In⁽³⁺⁾, La⁽³⁺⁾, Yb⁽³⁺⁾ und die entsprechenden Salze mit D(-)-N-Metylglucamin.

8. Verfahren zur Herstellung von Metallchelaten der Verbindungen der Formel I nach Anspruch 1, bei welchem ein Salz oder ein Metalloxid, ausgewählt aus solchen mit den Ordnungszahlen von 20 bis 31, 39, 42, 43, 44, 49 oder von 57 bis 83, mit einer Verbindung der Formel I nach Anspruch 1 reagiert wird, gegebenenfalls in Anwesenheit der für die Neutralisation benötigten Menge der Säure oder Base.

9. Diagnose-Präparat, bestehend aus
a) einem physiologisch akzeptablen Komplexsalz, bei welchem das ein Chelat bildende Mittel eine Verbindung der allgemeinen Formel I nach den Ansprüchen 1 bis 4 ist und das Metallion aus der Gruppe der Elemente ausgewählt ist, welche die Ordnungszahlen von 20 bis 31, 38, von 42 bis 44, 49 oder 57 bis 83 haben,
b) falls nötig, einem Ion einer anorganischen oder organischen Säure oder Base,
c) einem geeigneten physiologisch akzeptablen Träger,
d) eventuell physiologisch akzeptablen Additiven.

10. Diagnose-Präparat nach Anspruch 9, bei welchem das Komplexsalz in einer Konzentration von 0,002 bis 1 mol/l anwesend ist.

## Patentansprüche (Patentansprüche für folgende(n) Vertragsstaat(en): ES)

1. Verfahren zur Herstellung von 1,4,7,10-Tetraazacyclododecan der allgemeinen Formel I 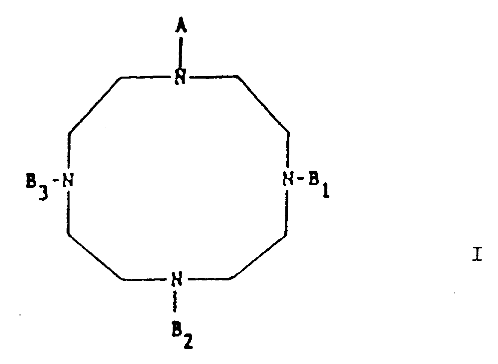 worin
A = eine Gruppe der Formel 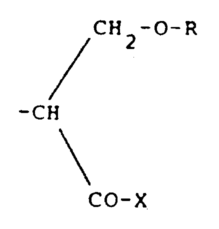
bei welcher
R = H oder eine C₁-C₅ gerade oder verzweigte Alkyl-Gruppe, oder eine Benzyl-Gruppe, die bei dem aromatischen Ring mono- oder substituiert werden kann durch Halogen-, Hydroxy-, Carboxy-, Carbamoyl-, Alkoxycarbonyl-, Sulphamoyl-, niedrige Alkyl-, niedrige Hydroxyalkyl-, Amino-, Acylamino-, Acyl-, Hydroxyacyl-Gruppen, oder eine Gruppe der Formel H(OCH₂CH₂)₁₋₄-, Me(OCH₂CH₂)₁₋₄-, oder Et(OCH₂CH₂)₁₋₄-,
X = eine O-R₁-Gruppe, bei welcher R₁ = H oder eine C₁-C₅ Alkyl-, Hydroxyalkyl-, Alkoxyalkyl-, Alkoxyhydroxyalkyl-Gruppe, oder eine Polyoxaalyl-Gruppe mit 1 bis 15 Sauerstoff-Atomen und 3 bis 45 Kohlenstoffatomen, oder X = eine -NR₂R₃-Gruppe, bei welcher R₂ und R₃, die gleich oder verschieden sein können, Wasserstoff, C₁-C₆ Alkyl-, Hydroxyalkyl-, Alkoxyalkyl- oder Alkoxyhydroxyalkyl-Gruppen mit bis zu 5 Hydroxy-Gruppen sind
und
B₁, B₂ und B₃, die gleich oder verschieden sein können, dieselbe Bedeutung haben wie A oder H oder eine Gruppe sind mit der Formel 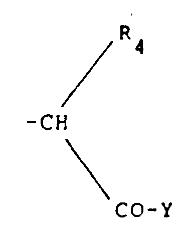 bei welcher
R₄ = H oder eine C₁-C₅ gerade oder verzweigte Alkyl-Gruppe,
Y = eine O-R₅-Gruppe, bei welcher R₅ = H oder eine C₁-C₅ Alkyl-, Hydroxyalkyl-, Alkoxyalkyl- oder Alkoxyhydroxyalkyl-Gruppe, oder eine Polyoxaalkyl-Gruppe mit 1 bis 15 Sauerstoffatomen und 3 bis 45 Kohlenstoffatomen, oder Y = eine -NR₆R₇-Gruppe, bei welcher R₆ und R₇, die gleich oder verschieden sein können, H oder C₁-C₆ Alkyl, Hydroxyalkyl-, Alkoxyalkyl- oder Alkoxyhydroxyalkyl-Gruppen sind mit bis zu 5 Hydroxy-Gruppen,
gekennzeichnet durch die folgenden Stufen:
a) Reaktion von 1,4,7,10-Tetraazacyclododecan (II) 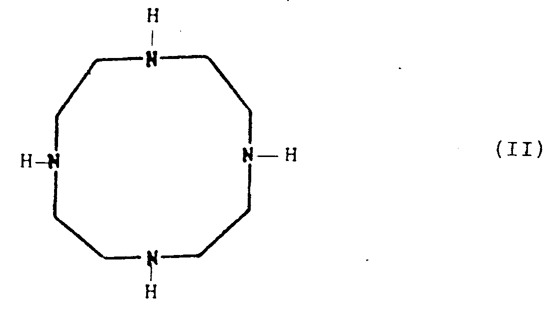 mit einem α-Halo-Propionyl-Derivat (III) 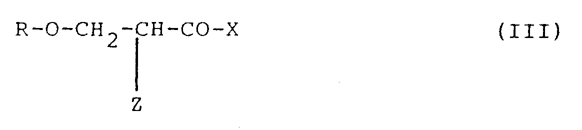 worin Z ein Halogen ist und R und X die oben definierten Bedeutungen haben, um das Additionsprodukt (IV) 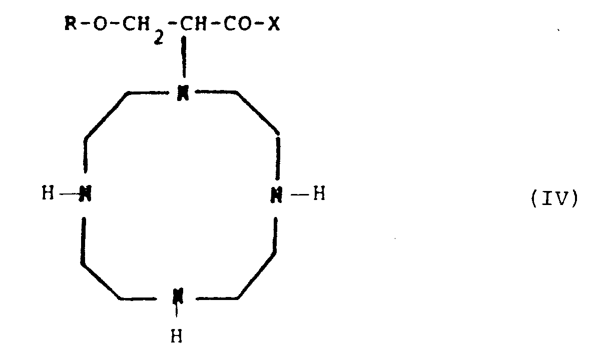 zu ergeben oder die korrespondierenden polysubstituierten Produkte an den Stickstoffatomen von II, abhängig von dem Überschuß von verwendetem III;
b) Kondensation der Verbindung (IV) oder der polysubstituierten Analoge davon mit dem α-Halo-Essigderivat (VIII), oder einem geeigneten Zwischenstoff davon 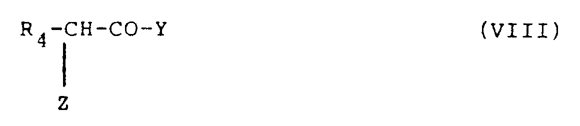 worin Z ein Halogen ist und R₄ und Y die oben definierten Bedeutungen haben, um die gewünschten Chelatmittel der allgemeinen Formel I zu erhalten.

2. Verfahren nach Anspruch 1, gekennzeichnet durch die folgenden Stufen:
a) Reaktion eines Diethylentriamins (V)
H₂N-CH₂CH₂-NH-CH₂CH₂-NH₂ (V)
das vorhorgehend mit einer geeigneten Schutzgruppe geschützt worden ist, mit dem Halo-Derivat (III), um die Verbindung (VII) zu erhalten 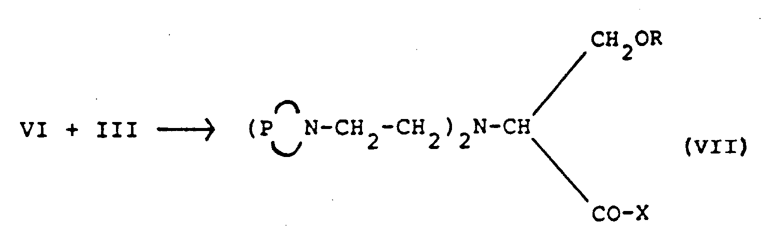 vorin P die Schutzgruppe ist, R und X wie oben definiert sind;
b) Eliminierung der Gruppe P von der Verbindung (VII);
c) Kondensation der befreiten Verbindung (VII) mit Tosyldiethanolamin, um die Verbindung (IV) zu erhalten;
d) Kondensation der Verbindung (IV) mit dem geeigneten Halo-Essigderivat (VIII) oder einem geeigneten Vorläufer davon.

3. Verfahren zur Herstellung von Metallchelaten der Verbindungen der Formel I nach Anspruch 1, bei welchem ein Salz oder ein Metalloxid, ausgewählt aus solchen mit den Ordnungszahlen von 20 bis 31, 39, 42, 43, 44, 49 oder von 57 bis 83, mit einer Verbindung der Formel I nach Anspruch 1 reagiert wird, gegebenenfalls in Anwesenheit der für die Neutralisation benötigten Menge der Säure oder Base.

## Revendications (Revendications pour l'(les) Etat(s) contractant(s) suivant(s): AT, BE, CH, DE, FR, GB, IT, LI, LU, NL, SE)

1. 1,4,7,10-tétraazacyclododécane de formule générale I 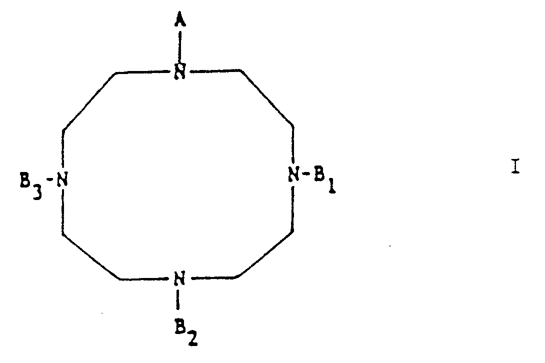 où
A est un groupe de formule 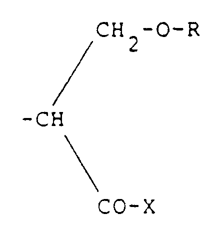
dans lequel
R est H ou un groupe alkyle linéaire ou ramifié en C₁-C₅, ou un groupe benzyle qui peut être mono- ou polysubstitué sur le noyau aromatique par des groupes halogéno, hydroxyle, carboxyle, carbamoyle, alcoxycarbonyles, sulfamyle, alkyles inférieurs, hydroxyalkyles inférieurs, amino, acylamino, acyles, hydroxyacyles, ou un groupe de formule H(OCH₂CH₂)₁₋₄-, Me(OCH₂CH₂)₁₋₄- ou Et(OCH₂CH₂)₁₋₄-,
X est un groupe O-R₁ dans lequel R₁ est H ou un groupe alkyle en C₁-C₅, hydroxyalkyle, alcoxyalkyle, alcoxyhydroxyalkyle, ou un groupe polyoxaalkyle ayant 1 à 15 atomes d'oxygène et 3 à 45 atomes de carbone, ou bien X est un groupe -NR₂R₃ dans lequel R₂ et R₃, qui peuvent être identiques ou différents, sont de l'hydrogène ou des groupes alkyles en C₁-C₆ , hydroxyalkyles, alcoxyalkyles ou alcoxyhydroxyalkyles ayant jusqu'à 5 groupes hydroxyle,
et
B₁, B₂ et B₃, qui peuvent être identiques ou différents, ont la même signification que A, ou bien ils sont H ou un groupe de formule 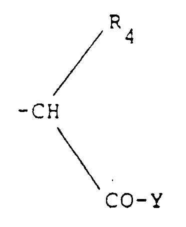 dans lequel
R₄ est H ou un groupe alkyle linéaire ou ramifié en C₁-C₅,
Y est un groupe O-R₅ dans lequel R₅ est H ou un groupe alkyle en C₁-C₅, hydroxyalkyle, alcoxyalkyle, alcoxyhydroxyalkyle, ou un groupe polyoxaalkyle ayant 1 à 15 atomes d'oxygène et 3 à 45 atomes de carbone, ou bien Y est un groupe -NR₆R₇ dans lequel R₆ et R₇, qui peuvent être identiques ou différents, sont H ou des groupes alkyles en C₁-C₆ hydroxyalkyles, alcoxyalkyles ou alcoxyhydroxyalkyles ayant jusqu'à 5 groupes hydroxyle,
lesdits dérivés étant, si nécessaire, salifiés par des bases organiques ou minérales appropriées, ainsi que les chélates du composé de formule I avec des ions métalliques, ces chélates pouvant, si nécessaire, être eux-mêmes neutralisés par des acides ou bases organiques ou minéraux.

2. Dérivés de 1,4,7,10-têtraazacyclododécane de formule générale II 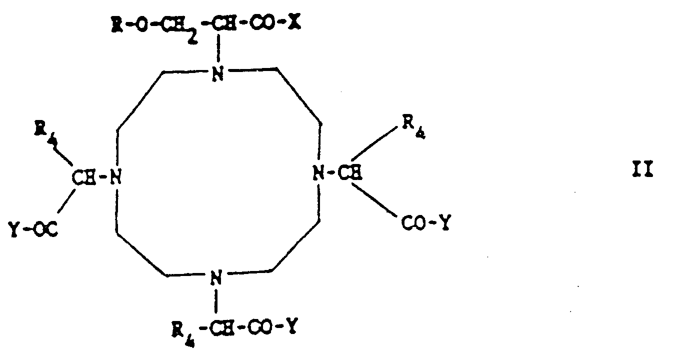 où
R, R₄, X et Y sont tels que définis dans la revendication 1, et leurs chélates avec des ions bi- ou trivalents d'éléments métalliques ayant des numéros atomiques de 20 à 31, 39, 42, 43, 44, 49 ou 57 à 83.

3. Dérivés de 1,4,7,10-tétraazacyclododécane de formule générale III 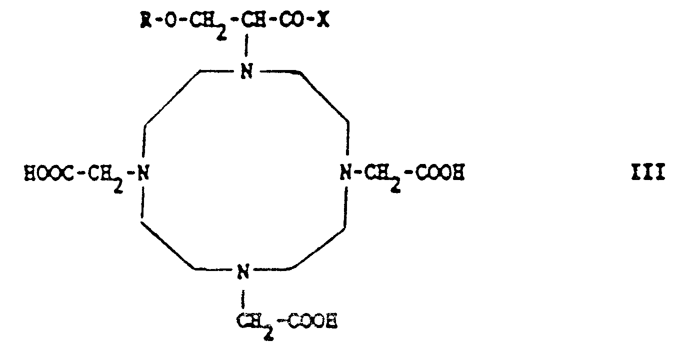 où R et X sont tels que définis dans la revendication 1, et leurs chélates avec des ions bi- ou trivalents d'éléments métalliques ayant des numéros atomiques de 20 à 31, 39, 42, 43, 44, 49 ou 57 à 83.

4. Dérivés de sérine de formule générale IV 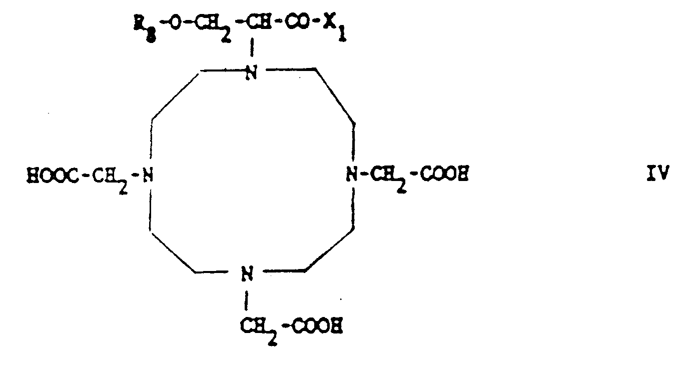 où R₈ est H ou le groupe benzyle, X₁ est OH, -NH₂, -NHCH₂CH₂OH, -NHCH(CH₂OH)₂, -NHCH₂CH(OH)CH₂OH, -N(CH₂CH₂OH)₂, -NH-CH₂-CH(OH)-CH₂OCH₃ ou -NH-CH₂-CH(OH)-CH(OH)-CH₂OH, et leurs chélates avec des ions bi- ou trivalents d'éléments métalliques ayant des numéros atomiques de 20 à 31, 38, 42, 43, 44, 49 ou 57 à 83.

5. Un chélate d'un composé tel que revendiqué dans les revendications 1 à 4, dans lequel l'ion métallique du chélate est Fe⁽²⁺⁾, Fe⁽³⁺⁾, Cu⁽²⁺⁾, Gd⁽³⁺⁾, Eu⁽³⁺⁾, Dy⁽³⁺⁾ ou Mn⁽²⁺⁾.

6. Chélates tels que revendiqués dans la revendication 2, formés avec les ions des radio-isotopes suivants : ⁵¹Cr, ⁶⁸Ga, ¹¹¹In, ^{99m}Tc, ¹⁴⁰La, ¹⁶⁸Yb.

7. Un composé tel que revendiqué dans les revendications 1 à 6, choisi dans le groupe formé par :
- l'acide 2-(1,4,7,10-tétraazacyclododécane-1-yl)-3-benzyloxypropionique,
- l'acide 2-(1,4,7,10-tétraazacyclododécane-1-yl)-3-hydroxypropionique,
- l'acide 2-[1,4,7,10-tétraaza-4-(1-carboxy-2-benzyloxyéthyl)cyclododécane-1-yl]-3-benzyloxypropionique,
- l'acide 2-[1,4,7,10-tétraaza-4-(1-carboxy-2-hydroxyéthyl)cyclododécane-1-yl]-3-hydroxypropionique,
- l'acide 2-[1,4,7,10-tétraaza-7-(1-carboxy-2-benzyloxyéthyl)cyclododécane-1-yl]-3-benzyloxypropionique,
- l'acide 2-[1,4,7,10-tétraaza-7-(1-carboxy-2-hydroxyéthyl)cyclododécane-1-yl]-3-hydroxypropionique,
- l'acide 2-[1,4,7,10-tétraaza-4,7-di(1-carboxy-2-benzyloxyéthyl)cyclododécane-1-yl]-3-benzyloxypropionique,
- l'acide 2-[1,4,7,10-tétraaza-4,7-di(1-carboxy-2-hydroxyéthyl)cyclododécane-1-yl]-3-hydroxypropionique,
- l'acide 2-[1,4,7,10-tétraaza-4-(1-carboxy-2-benzyloxyéthyl)-7,10-di(carboxyméthyl)cyclododécane-1-yl]-3-benzyloxypropionique,
- l'acide 2-[1,4,7,10-tétraaza-4-(1-carboxy-2-hydroxyéthyl)-7,10-di(carboxyméthyl)cyclododécane-1-yl]-3-hydroxypropionique,
- l'acide 2-[1,4,7,10-tétraaza-7-(1-carboxy-2-benzyloxyéthyl)-4,10-di(carboxyméthyl)cyclododécane-1-yl]-3-benzyloxypropionique,
- l'acide 2-[1,4,7,10-tétraaza-7-(1-carboxy-2-hydroxyéthyl)-4,10-di(carboxyméthyl)cyclododécane-1-yl]-3-hydroxypropionique,
- l'acide 2-[1,4,7,10-tétraaza-4,7-di(1-carboxy-2-benzyloxyéthyl)-10-carboxyméthylcyclododécane-1-yl]-3-benzyloxypropionique,
- l' acide 2-[1,4,7,10-tétraaza-4,7-di(1-carboxy-2-hydroxyéthyl)-10-carboxyméthylcyclododécane-1-yl]-3-hydroxypropionique,
- l'acide 2-[1,4,7,10-tétraaza-4-7,10-tri(carboxyméthyl)cyclododécane-1-yl]-3-benzyloxypropionique,
- l'acide 2-[1,4,7,10-tétraaza-4,7,10-tri(carboxyméthyl)cyclododécane-1-yl]-3-hydroxypropionique,
- le 2-(1,4,7,10-tétraazacyclododécane-1-yl)-3-benzyloxypropionamide,
- le 2-(1,4,7,10-tétraazacyclododécane-1-yl)-3-hydroxypropionamide,
- le2-(1,4,7,10-tétraazacyclododécane-1-yl)-3-benzyloxy-N-(2-hydroxyéthyl)propionamide,
- le 2-(1,4,7,10-tétraazacyclododécane-1-yl)-3-hydroxy-N-(2-hydroxyéthyl)propionamide,
- le2-(1,4,7,10-tétraazacyclododécane-1-yl)-3-benzyloxy-N-(1,3-dihydroxyisopropyl)propionamide,
- le 2-(1,4,7,10-tétraazacyclododécane-1-yl)-3-hydroxy-N-(1,3-dihydroxyisopropyl)propionamide,
- le2-(1,4,7,10-tétraazacyclododécane-1-yl)-3-benzyloxy-N-(2,3-dihydroxypropyl)propionamide,
- le 2-(1,4,7,10-tétraazacyclododécane-1-yl)-3-hydroxy-N-(2,3-dihydroxypropyl)propionamide,
- le 2-(1,4,7,10-tétraazacyclododécane-1-yl)-3-benzyloxy-N,N-di(2-hydroxyéthyl)propionamide,
- le2-(1,4,7,10-tétraazacyclododécane-1-yl)-3-hydroxy-N,N-di(2-hydroxyéthyl)propionamide,
- le 2-[1,4,7,10-tétraaza-4,7,10-tri(carboxyméthyl)cyclododécane-1-yl]-3-benzyloxypropionamide,
- le 2-[1,4,7,10-tétraaza-4,7,10-tri(carboxyméthyl)cyclododécane-1-yl]-3-hydroxypropionamide,
- le 2-[1,4,7,10-tétraaza-4,7,10-tri(carboxyméthyl)cyclododécane-1-yl]-3-benzyloxy-N-(2-hydroxyéthyl)propionamide,
- le 2-[1,4,7,10-tétraaza-4,7,10-tri(carboxyméthyl)cyclododécane-1-yl]-3-hydroxy-N-(2-hydroxyéthyl)propionamide,
- le 2-[1,4,7,10-tétraaza-4,7,10-tri(carboxyméthyl)cyclododécane-1-yl]-3-benzyloxy-N-(1,3-dihydroxyisopropyl)propionamide,
- le 2-[1,4,7,10-tétraaza-4,7,10-tri(carboxyméthyl)cyclododécane-1-yl]-3-hydroxy-N-(1,3-dihydroxyisopropyl)propionamide,
- le 2-[1,4,7,10-tétraaza-4,7,10-tri(carboxyméthyl)cyclododécane-1-yl]-3-benzyloxy-N-(2,3-dihydroxypropyl)propionamide,
- le 2-[1,4,7,10-tétraaza-4,7,10-tri(carboxyméthyl)cyclododécane-1-yl]-3-hydroxy-N-(2,3- dihydroxypropyl)propionamide,
- le 2-[1,4,7,10-tétraaza-4,7,10-tri(carboxyméthyl)cyclododécane-1-yl]-3-benzyloxy-N,N-di(2-hydroxyéthyl)propionamide,
- le 2-[1,4,7,10-tétraaza-4,7,10-tri(carboxyméthyl)cyclododécane-1-yl]-3-hydroxy-N,N-di(2-hydroxyéthyl)propionamide,
les complexes chélatés respectifs formés avec Fe⁽³⁺⁾, Cu⁽²⁺⁾, Mn⁽²⁺⁾, Gd⁽³⁺⁾, Dy⁽³⁺⁾, In⁽³⁺⁾, La⁽³⁺⁾, Yb⁽³⁺⁾ et les sels correspondants formés avec la D(-)-N-méthylglucamine.

8. Un procédé pour la préparation de chélates métalliques des composés de formule I de la revendication 1, qui consiste à faire réagir un sel ou un oxyde de métal choisi parmi ceux ayant les numéros atomiques de 20 à 31, 39, 42, 43, 44, 49 ou 57 à 83 avec un composé de formule I de la revendication 1, éventuellement en présence de la quantité d'acide ou de base nécessaire à la neutralisation.

9. Une préparation à usage diagnostique comprenant a) un sel complexe physiologiquement acceptable, dans lequel l'agent chélateur est un composé de formule générale I selon les revendications 1 à 4 et l'ion métallique est choisi dans le groupe des éléments ayant les numéros atomiques de 20 à 31, 38, 42 à 44, 49 ou 57 à 83, b) si nécessaire, un ion d'un acide ou d'une base organique ou minéral, c) un support approprié physiologiquement acceptable, d) facultativement, des additifs physiologiquement acceptables.

10. Une préparation à usage diagnostique selon la revendication 9, dans laquelle le sel complexe est présent à une concentration de 0,002 à 1 mol/ℓ.

## Revendications (Revendications pour l'(les) Etat(s) contractant(s) suivant(s): ES)

1. Un procédé de préparation d'un 1,4,7,10-tétraazacyclododécane de formule générale I 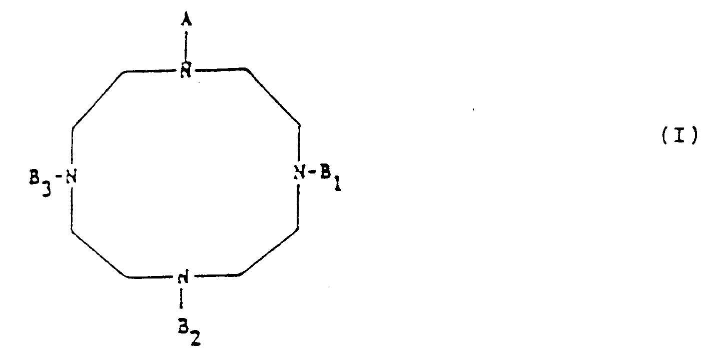 où
A est un groupe de formule 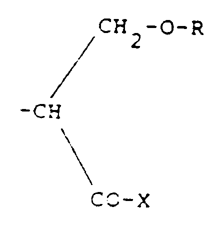
dans lequel
R est H ou un groupe alkyle linéaire ou ramifié en C₁-C₅, ou un groupe benzyle qui peut être mono- ou polysubstitué sur le noyau aromatique par des groupes halogéno, hydroxyle, carboxyle, carbamoyle, alcoxycarbonyles, sulfamyle, alkyles inférieurs, hydroxyalkyles inférieurs, amino, acylamino, acyles, hydroxyacyles, ou un groupe de formule H(OCH₂CH₂)₁₋₄-, Me(OCH₂CH₂)₁₋₄- ou Et OCH₂CH₂)₁₋₄-,
X est un groupe O-R₁ dans lequel R₁ est H ou un groupe alkyle en C₁-C₅, hydroxyalkyle, alcoxyalkyle, alcoxyhydroxyalkyle, ou un groupe polyoxaalkyle ayant 1 à 15 atome d'oxygène et 3 à 45 atomes de carbone, ou bien X est un groupe -NR₂R₃ dans lequel R₂ et R₃, qui peuvent être identiques ou différents, sont de l'hydrogène ou des groupes alkyles en C₁-C₆ , hydroxyalkyles, alcoxyalkyles ou alcoxyhydroxyalkyles ayant jusqu'à 5 groupes hydroxyle,
et
B₁, B₂ et B₃, qui peuvent être identiques ou différents, ont la même signification que A, ou bien ils sont H ou un groupe de formule 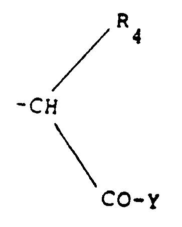 dans lequel
R₄ est H ou un groupe alkyle linéaire ou ramifié en C₁-C₅,
Y est un groupe O-R₅ dans lequel R₅ est H ou un groupe alkyle en C₁-C₅, hydroxyalkyle, alcoxyalkyle, alcoxyhydroxyalkyle, ou un groupe polyoxaalkyle ayant 1 à 15 atomes d'oxygène et 3 à 45 atomes de carbone, ou bien Y est un groupe -NR₆R₇ dans lequel R₆ et R₇, qui peuvent être identiques ou différents, sont H ou des groupes alkyles en C₁-C₆, hydroxyalkyles, alcoxyalkyles ou alcoxyhydroxyalkyles ayant jusqu'à 5 groupes hydroxyle,
caractérisé par les étapes suivantes :
a) réaction du 1,4,7,10-tétraazacyclododécane (II) 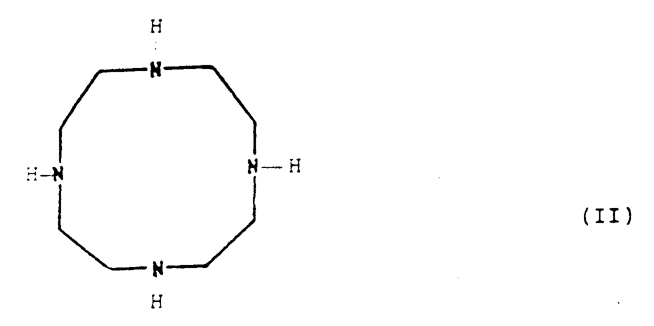 avec un dérivé α-halogéno-propionylé III 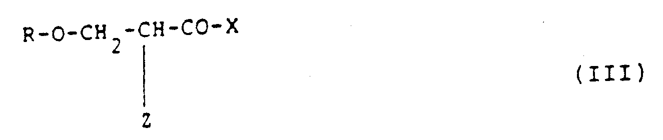 où Z est un halogène et R et X sont tels que définis ci-dessus, pour donner le produit d'addition IV 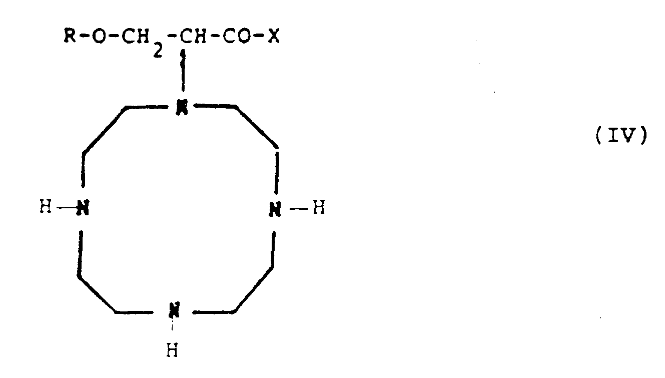 ou les produits polysubstitués correspondants sur les atomes d'azote de II, selon l'excès de III utilisé;
b) condensation du composé IV ou de ses analogues polysubstitués avec le dérivé d'halogéno-acétique VIII, ou un précurseur appliqué de celui-ci, 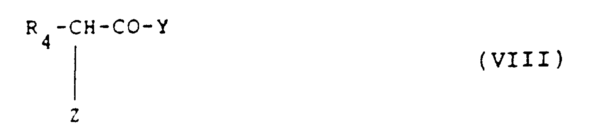 où Z est un halogène et R₄ et Y sont tels que définis ci-dessus, pour donner les agents chélatants désirés de formule générale I.

2. Un procédé selon la revendication 1, caractérisé par les étapes suivantes :
a) réaction d'une diéthylènetriamine (V)
H₂N-CH₂CH₂-NH-CH₂CH₂-NH₂ (V)
qui a été préalablement protégée par un groupe protecteur approprié, avec le dérivé halogéné (III) pour donner un composé (VII) 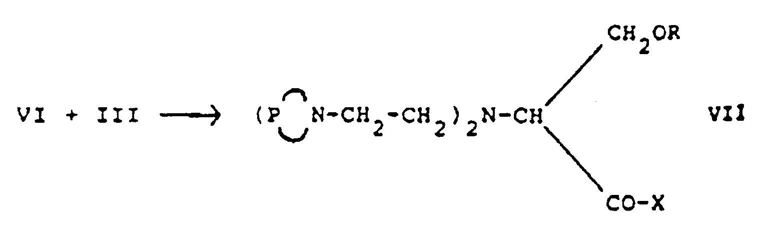 où P est ledit groupe protecteur, R et X sont tels que définis ci-dessus ;
b) élimination du groupe P du composé (VII) ;
c) condensation du composé (VII) déprotégé avec de la tosyldiéthanolamine pour donner le composé (IV) ;
d) condensation du composé (IV) avec le dérivé hagéno-acétique (VIII) approprié ou un précurseur approprié de celui-ci.

3. Un procédé pour la préparation de chélates métalliques des composés de formule I de la revendication 1, qui consiste à faire réagir un sel ou un oxyde de métal choisi parmi ceux ayant les numéros atomiques de 20 à 31, 39, 42, 43, 44, 49 ou 57 à 83 avec un composé de formule I de la revendication 1, éventuellement en présence de la quantité d'acide ou de base nécessaire à la neutralisation.
